# FASCICULE DE BREVET EUROPEEN

(11) **EP 2 852 582 B1**
(45) Date de publication et mention de la délivrance du brevet: **20.07.2016**
(21) Numéro de dépôt: 13724829.0
(22) Date de dépôt: 23.05.2013
(51) Int. Cl.: C07D 307/56, C07D 307/58, C07D 307/64, C07D 307/68, A01N 43/08

(54) **NOUVEAUX ANALOGUES DE STRIGOLACTONE ET LEUR UTILISATION POUR LE TRAITEMENT DES PLANTES**
NEUE ANALOGA VON STRIGOLACTON UND DEREN VERWENDUNG IN DER PFLANZENBEHANDLUNG
NEW ANALOGS OF STRIGOLACTONE AND THEIR USE FOR TREATING PLANTS

(30) Priorité: 23.05.2012 FR 1254700
(43) Date de publication de la demande: 01.04.2015
(73) Titulaire: Institut National de la Recherche Agronomique (INRA), 75007 Paris (FR); Centre National de la Recherche Scientifique (CNRS), 75016 Paris (FR); Universite De Paris 11-Paris Sud, 91400 Orsay (FR)
(72) Inventeur: BOYER, François-Didier, F-78150 Le Chesnay (FR); RAMEAU, Catherine, F-75015 Paris (FR); PILLOT, Jean-Paul, F-78000 Versailles (FR); SERVAJEAN, Vincent, F-92360 Meudon La Foret (FR); DE SAINT GERMAIN, Alexandre, 92037 La Jolla (US); BEAU, Jean-Marie, F-45240 Menestreau En Vilette (FR); POUVREAU, Jean-Bernard, F-44300 Nantes (FR); CLAVE, Guillaume, F-78990 Elancourt (FR)
(74) Mandataire: Ipside
(86) Numéro de dépôt international: PCT/EP2013/060624
(87) Numéro de publication internationale: WO 2013/174925

(56) Documents cités:
- DATABASE CAPLUS [Online] CHEMICAL ABSTRACTS SERVICE, COLUMBUS, OHIO, US; YAMAGUCHI, SHINJIRO ET AL: "Plant branching inhibitor, method for producing same, and plant branching inhibitory composition", XP002686074, extrait de STN Database accession no. 2010:1500232 -& WO 2010/137662 A1 (RIKEN CORP., JAPAN; OSAKA PREFECTURE UNIVERSITY PUBLIC CORPORATION) 2 décembre 2010 (2010-12-02)
- DATABASE CAPLUS [Online] CHEMICAL ABSTRACTS SERVICE, COLUMBUS, OHIO, US; ASAMI, TADAO ET AL: "Preparation of 2,5-dihydrofuran-2-one derivatives having strigolactone-like activity", XP002686075, extrait de STN Database accession no. 2012:499394 -& WO 2012/043813 A1 (RIKEN CORP., JAPAN) 5 avril 2012 (2012-04-05)

## Description

La présente invention concerne de nouveaux composés aptes à contrôler la ramification des plantes supérieures, une composition comportant de tels composés, et leur utilisation pour le traitement des plantes supérieures, notamment en vue de contrôler leur ramification, par une inhibition sélective ou globale de la croissance de bourgeons sur la plante. Cette inhibition peut être temporaire, de manière à contrôler la période de développement de ces bourgeons, ou permanente, afin par exemple de favoriser la croissance d'autres parties de la plante au détriment de celle(s) inhibée(s).

Les composés selon l'invention trouvent notamment des applications dans le domaine agricole, pour la culture de plantes, telles que des plantes alimentaires, des légumineuses, des plantes forestières, des plantes ornementales etc., pour lesquelles le contrôle du nombre de ramifications, du démarrage de bourgeons et/ou de la période de ramification peut améliorer le rendement et/ou la qualité de la production (taille du fruit, qualité du bois, etc.). Par plantes supérieures, on entend les végétaux pluricellulaires, vasculaires, munis de racines et d'une partie aérienne. Par culture, on entend aussi bien la culture en champ, qu'en plantation pour les forêts, que la culture in vitro, hors sol ou autre.

Les plantes cultivées, que ce soit pour leurs fleurs, leurs fruits, leurs graines ou pour leurs parties végétatives, font l'objet de nombreux contrôles et traitements, de manière à obtenir le meilleur rendement possible et la meilleure qualité.

Ainsi par exemple, on essaie de maîtriser les périodes de floraison de manière à éviter que les bourgeons floraux soient initiés pendant les périodes de forts risques de gel. De même, lorsqu'on souhaite obtenir des fruits de gros calibre, ou plus généralement des plantes plus vigoureuses, on procède à une taille de la plante de manière à limiter le nombre de ramifications et ainsi le nombre d'organes « puits » que sont les fruits en période de grossissement ou les graines en cours de remplissage. L'utilisation d'engrais permet également d'optimiser les rendements.

De tels contrôles et traitements nécessitent une connaissance, outre de la plante elle-même, des conditions dans lesquelles elle est cultivée : nature du sol, climat, etc., notamment pour savoir quand et comment tailler les plantes. Par ailleurs, la taille est un procédé manuel fastidieux, coûteux nécessitant l'intervention de personnes qualifiées.

Pour remédier à ces inconvénients, il a été proposé par l'art antérieur des procédés de traitement chimique des plantes pour contrôler leur croissance, par inhibition totale ou partielle, définitive ou temporaire, de la croissance des ramifications, de manière notamment à optimiser le rendement de ces plantes.

En particulier, il a été proposé dans le document de brevet FR-A-2 930 402 de mettre les plantes à traiter en contact avec une solution contenant une strigolactone, naturelle ou de synthèse, de manière à inhiber ou limiter la pousse de tout ou partie des ramifications.

Les strigolactones sont des hormones végétales de la famille des apocaroténoïdes. Elles sont principalement composées d'un squelette à quatre cycles dits A, B, C, D, plus précisément d'une lactone tricyclique ABC connectée par une liaison éther d'énol à un cycle butyrolactone dit cycle D. On connaît actuellement de nombreuses strigolactones naturelles, telles que la sorgolactone, le 5-désoxy-strigol, le strigol, l'orobanchol, le 2' epi-orobanchol, le solanacol, l'acétate d'orobanchyle ou l'acétate de strigyle, et des strigolactones de synthèse, telles que le GR24 ou le GR5. Des applications des strigolactones ont été décrites non seulement pour le contrôle de la croissance et de l'architecture des plantes supérieures, mais également pour induire la germination des graines de plantes parasites telles que les *Orobanches.* Afin d'éliminer de telles plantes parasites des sols agricoles, il est ainsi proposé de traiter lesdits sols avec des strigolactones, de manière à induire la germination des plantes parasites en l'absence de plantes hôtes indispensables à leur existence, ce qui entraîne leur mort.

Parmi les strigolactones, la molécule dite GR24, de formule : 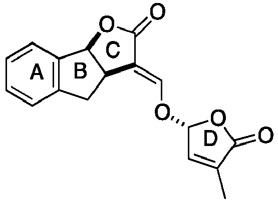 dans laquelle le cycle A est un cycle aromatique,
et la molécule dite GR5, de formule : 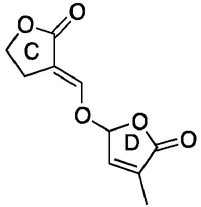 dépourvue de cycles A et B,
ont été décrites par l'art antérieur comme particulièrement efficaces pour réprimer la ramification des plantes supérieures. Ces composés, outre le fait d'être peu faciles à synthétiser, présentent cependant l'inconvénient d'être relativement cytotoxiques.

La présente invention vise à proposer de nouveaux composés susceptibles d'être mis en oeuvre dans des procédés de traitement des plantes, qui soient au moins aussi efficaces que les strigolactones naturelles, et de préférence que les strigolactones synthétiques proposées par l'art antérieur, en terme de contrôle du degré de ramification des plantes, tout en étant plus simples à synthétiser et en présentant notamment une cytotoxicité moindre.

A cet effet, il est proposé selon l'invention de nouveaux composés répondant à la formule générale (I) : 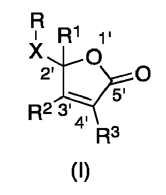 dans laquelle :
X représente un atome d'oxygène, un atome de soufre, NH ou un radical N-alkyl,
R¹ et R², identiques ou différents, représentent chacun un atome d'hydrogène ou un radical hydrocarboné linéaire ou ramifié, saturé ou insaturé, en C1-C10, R¹ et R² ne représentant pas tous deux un atome d'hydrogène,
R³ représente un radical hydrocarboné linéaire ou ramifié, saturé ou insaturé, en C1-C10,
et R représente un radical phényle mono-substitué par un substituant Y choisi parmi Cl, Br, I et CF₃, ou un radical phényle di-substitué par un substituant Y et un substituant Z, Y et Z, identiques ou différents, étant choisis chacun parmi Cl, Br, I et CF₃, ou formant ensemble un cycle saturé ou insaturé ou aromatique, contenant le cas échéant un ou plusieurs hétéroatomes, éventuellement substitué, notamment un cycle à 5 à 8 atomes, de préférence à 6 atomes,
ou R représente un radical : 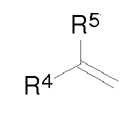
où R⁴ représente un radical hydrocarboné linéaire ou ramifié, saturé ou insaturé,
et R⁵ représente un radical hydrocarboné linéaire ou ramifié, saturé ou insaturé, le cas échéant substitué, un groupe COR⁶ ou un groupe CO₂R⁶, où R⁶ représente un atome d'hydrogène ou un radical hydrocarboné linéaire ou ramifié, saturé ou insaturé

Toute forme isomère de ces composés sur le carbone C₂, (stéréo-isomères 2'α et 2'β) et tout mélange de telles formes isomères entre également dans le cadre de l'invention. A partir d'un mélange d'isomères, chaque isomère peut être obtenu par des méthodes classiques en elles-mêmes pour l'homme du métier.

Dans la formule (I) ci-dessus, R₄ et R₅ sont en outre interchangeables.

On entend dans la présente description, par radical phényle mono-substitué, un radical phényle substitué par un substituant ou plus, c'est-à-dire un radical phényle substitué par au moins un substituant Y. De même, on entend par radical phényle di-substitué, un radical phényle substitué par deux substituants ou plus, c'est-à-dire un radical phényle substitué par au moins un substituant Y et un substituant Z.

Il a été découvert par les présents inventeurs que ces composés particuliers, analogues des strigolactones en ce qui concerne le cycle D, inédits à ce jour, étaient particulièrement actifs pour la répression de la ramification des plantes. Leur efficacité, bien supérieure à celle des strigolactones naturelles, est comparable à celle des autres composés analogues synthétiques proposés par l'art antérieur, en particulier le GR24 et le GR5.

En particulier, les présents inventeurs ont découvert que les analogues des strigolactones répondant à la formule (I) étaient capables d'inhiber le démarrage des bourgeons axillaires, notamment chez le pois (*Pisum sativum L.*), à une concentration inférieure à celle des strigolactones naturelles.

Les composés selon l'invention sont en outre particulièrement faciles à préparer, notamment en comparaison avec les analogues de strigolactones à 4 cycles. Ils présentent également avantageusement une stabilité en milieu aqueux bien supérieure à celle des strigolactones naturelles, qui de manière inhérente sont instables dans l'eau, en particulier à pH supérieur à 7, mais aussi à celle du GR24 et du GR5. Leur cytotoxicité est en outre avantageusement bien inférieure à celle de ces composés synthétiques GR24 et GR5.

De manière particulièrement avantageuse, les composés selon l'invention n'agissent en outre que faiblement sur la germination des graines des plantes parasites, notamment du type orobanche. En particulier, ils présentent une activité au moins 10 à 100 000 fois moins importante que les composés GR24 et GR5 pour la germination des graines d'orobanches rameuses (*Phelipanche ramosa, Orobanche minor* et *Orobanche cumana*) et de *Striga hermonthica.* La pullulation de telles plantes parasites nuisibles s'avérant un problème de plus en plus présent tant dans les régions tempérées que dans les régions tropicales, on comprend aisément l'intérêt de disposer de moyens de traitement des cultures végétales ne favorisant pas la germination des graines de ces plantes parasites, contrairement aux strigolactones naturelles ou à la plupart de leurs analogues de synthèse décrits par l'art antérieur, qui quant à elles l'induisent.

Selon une caractéristique préférée des composés selon l'invention, R³ représente un radical alkyle linéaire en C1-C10, de préférence un radical méthyle.

R¹ et R² représentent en outre chacun de préférence un atome d'hydrogène ou un radical alkyle linéaire ou ramifié en C1-C10.

De manière encore préférée, R¹ représente un atome d'hydrogène et R² représente un radical alkyle linéaire ou ramifié en C1-C10, de préférence un radical méthyle.

Préférentiellement, X représente un atome de soufre.

Dans des modes de réalisation particuliers de l'invention, R représente un radical phényle substitué au moins en position para.

Un composé particulièrement préféré selon l'invention répond à la formule générale (II) : 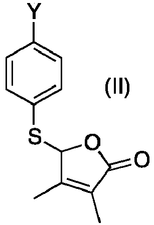 dans laquelle Y est choisi parmi CI, Br, I et CF₃, et dans laquelle X est un atome de soufre, R¹ est un atome d'hydrogène, R² et R³ sont tous deux des radicaux méthyles, et R est un radical phényle mono-substitué par le substituant Y en position para.

Parmi les composés répondant à la formule générale (II) ci-dessus, on peut citer en particulier le composé dans lequel Y est un atome de chlore, répondant à la formule générale (IIa) ci-dessous, le composé dans lequel Y est un atome de brome, répondant à la formule générale (IIb) ci-dessous, et le composé dans lequel Y représente CF₃, répondant à la formule (IIc) ci-dessous :

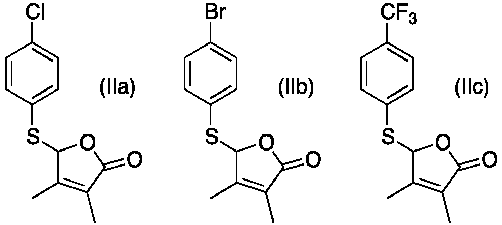

Un autre composé selon l'invention répond à la formule générale (II') : 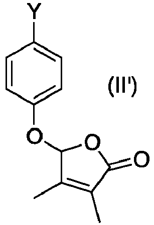 dans laquelle Y est choisi parmi CI, Br, I et CF₃, et dans laquelle X est un atome d'oxygène, R¹ est un atome d'hydrogène, R² et R³ sont tous deux des radicaux méthyles, et R est un radical phényle mono-substitué par le substituant Y en position para.

D'autres composés particuliers selon l'invention sont tels que R représente un radical phényle di-substitué de type coumarine, de formule : 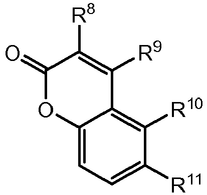 dans laquelle R⁸, R⁹, R¹⁰ et R¹¹, identiques ou différents, représentent chacun un atome d'hydrogène, CI, Br, I, CF₃, CHO, CN, NO₂, un radical hydrocarboné linéaire ou ramifié, saturé ou insaturé, le cas échéant substitué, ou un groupement CO₂R¹², où R¹² représente un atome d'hydrogène ou un radical hydrocarboné linéaire ou ramifié, saturé ou insaturé, de préférence en C1-C10.

De tels composés répondent ainsi à la formule générale (II") ci-après : 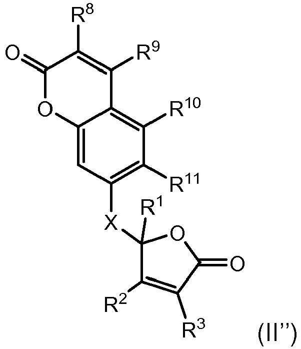 dans laquelle :
X représente un atome d'oxygène, un atome de soufre, NH ou un radical N-alkyl,
R¹ et R², identiques ou différents, représentent chacun un atome d'hydrogène ou un radical hydrocarboné linéaire ou ramifié, saturé ou insaturé, en C1-C10, R¹ et R² ne représentant pas tous deux un atome d'hydrogène,
R³ représente un radical hydrocarboné linéaire ou ramifié, saturé ou insaturé, en C1-C10,
R⁸, R⁹, R¹⁰ et R¹¹ sont tels que définis ci-dessus.

Plus particulièrement, des composés avantageux selon l'invention répondent à la formule générale (II"') : 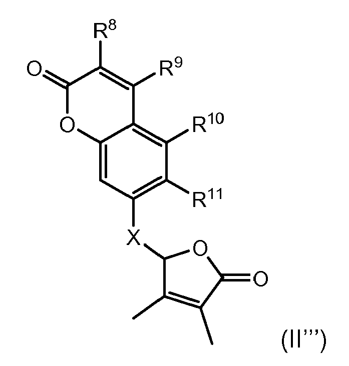 dans laquelle X, R⁸, R⁹, R¹⁰ et R¹¹ sont tels que définis ci-dessus.

Un composé particulier selon l'invention est tel que R⁸, R⁹, R¹⁰ et R¹¹ représentent tous chacun un atome d'hydrogène.

Dans des modes de réalisation différents de l'invention, R représente un radical : 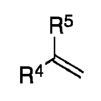
où R⁴ représente un radical alkyle ou un radical alkényle linéaires ou ramifiés en C1-C15,
et R⁵ représente un radical hydrocarboné linéaire ou ramifié, saturé ou insaturé, en C1-C10, de préférence en C1-C5, le cas échéant substitué, un groupe COR⁶ ou un groupe CO₂R⁶, où R⁶ représente un radical hydrocarboné linéaire ou ramifié en C1-C10, de préférence en C1-C5.

Entrent ainsi dans le cadre de l'invention les composés de formule générale (III) : 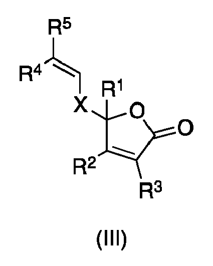 dans laquelle R¹, R², R³, R⁴ et R⁵ sont tels que définis ci-avant.

Des composés particuliers selon l'invention répondent à la formule générale (III) ci-avant, dans laquelle R⁴ représente un radical alkyle ou un radical alkényle linéaires ou ramifiés, de préférence en C1-C15, et R⁵ représente un radical hydrocarboné linéaire ou ramifié, de préférence insaturé, en C1-C10, de préférence en C1-C5, substitué, préférentiellement à son extrémité libre, par un groupement électro-attracteur. Des exemples de tels groupements électro-attracteurs sont CHO, CN, NO₂, CO₂R⁷, où R⁷ représente un radical hydrocarboné linéaire ou ramifié en C1-C10, de préférence en C1-C5.

Les composés de formule (III) ci-dessus dans lesquels R³ représente un radical méthyle, s'avèrent particulièrement avantageux du point de vue de l'efficacité d'inhibition du démarrage des bourgeons axillaires. Avantageusement, R¹ représente en outre un atome d'hydrogène et/ou R² représente également un radical méthyle.

Des composés répondant à la formule générale (III') ci-après s'avèrent ainsi particulièrement avantageux : 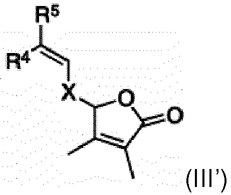 et plus particulièrement les composés de formule générale (III") ci-après : 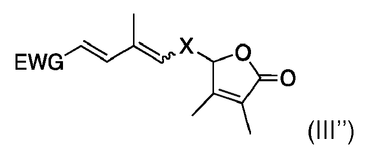 dans laquelle EWG représente un groupement électro-attracteur choisi parmi CHO, CN, NO₂ et CO₂R⁷, où R⁷ représente un radical hydrocarboné linéaire ou ramifié en C1-C10, de préférence en C1-C5
et X représente O, NH ou S.

Les composés selon l'invention peuvent être obtenus par toute voie de synthèse adéquate. L'homme du métier saura en déterminer les étapes, les conditions opératoires et les précurseurs à mettre en oeuvre en fonction de la structure particulière du composé de formule générale (I) visé.

Un procédé de synthèse des composés selon l'invention répondant aux formules générales (II), (II') ou (III') ci-dessus peut répondre au schéma réactionnel général suivant : 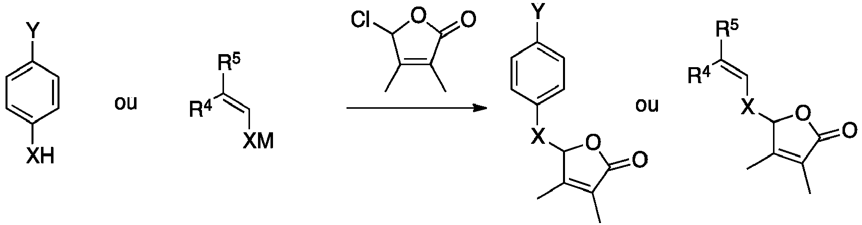 dans lequel M représente un métal alcalin, par exemple un atome de sodium ou de potassium.

Tout autre mode de synthèse que l'homme du métier saura identifier à partir de ses connaissances générales entre également dans le cadre de l'invention.

Un autre aspect de l'invention est une composition comprenant un composé tel que défini ci-avant, utilisable en particulier pour le traitement des plantes supérieures en vue de contrôler leur croissance et leur architecture, plus particulièrement leur ramification.

Préférentiellement, la concentration en ledit composé dans cette composition est comprise entre 0,1 nM et 10µM, préférentiellement entre 0,1 et 1000 nM, préférentiellement encore entre 1 et 100 nM.

Un troisième aspect de l'invention est l'utilisation d'un composé tel que défini ci-avant, et/ou d'une composition le comprenant, pour le traitement d'une plante supérieure en vue de contrôler sa croissance et son architecture.

Plus particulièrement, on place au contact de la plante une quantité adaptée d'un composé conforme à l'invention de manière à inhiber la formation d'au moins une ramification.

Par inhiber, on entend dans la présente description réprimer définitivement ou temporairement la croissance d'un bourgeon. Ainsi, selon l'invention, on peut supprimer une ramification en inhibant définitivement la croissance du bourgeon correspondant, ou mettre en dormance ledit bourgeon de manière à retarder dans le temps sa croissance.

Par ramification, on entend l'excroissance issue du bourgeon axillaire situé à l'aisselle des feuilles, que ce soit une branche, une fleur ou une inflorescence.

L'inhibition peut être globale, c'est-à-dire toucher tous les bourgeons axillaires au moment du traitement de la plante, ou ciblée, c'est-à-dire ne toucher que des bourgeons spécifiquement visés par le traitement.

Les plantes traitées peuvent aussi bien être cultivées en serres, qu'en champs, in vitro ou même hors sol.

Une quantité adaptée s'entend d'une quantité au moins suffisante pour agir sur la croissance et l'architecture de la plante à traiter. Cette quantité est évaluée pour chaque cas particulier, en fonction notamment de la nature de la plante à traiter et du degré de contrôle de la ramification souhaité, par exemple selon que l'on souhaite inhiber définitivement ou temporairement la croissance du bourgeon.

Selon l'invention, on peut appliquer une composition comportant un composé conforme à l'invention sur une portion au moins partielle de la partie aérienne de la plante. Par exemple, on peut appliquer, par vaporisation ou par dépôt, ladite composition sur des bourgeons axillaires de la plante que l'on souhaite réprimer, de manière à contrôler la croissance des bourgeons ainsi traités, ou plus généralement sur la partie de la plante dont on souhaite contrôler la croissance. Il est autrement possible d'injecter la composition comprenant un composé conforme à l'invention dans une partie aérienne de ladite plante, par exemple au niveau des bourgeons même, ou des tiges portant les bourgeons à réprimer, de manière à contrôler la croissance de la plante située au-dessus de la zone d'injection.

Dans un autre mode de mise en oeuvre, l'invention prévoit d'apporter une composition comprenant le composé conforme à l'invention par au moins une racine de ladite plante, de manière à contrôler la ramification et/ou la hauteur de la plante. Ceci peut-être réalisé par un enrichissement du sol en composé selon l'invention, de manière à diminuer de manière non sélective le nombre de tiges ou de freiner leur croissance. En effet, les inventeurs ont observé que le signal de répression SMS de la croissance des ramifications migre dans le sens racine - tige, ce qui laisse penser qu'il est véhiculé par la sève brute du xylème.

Avantageusement, la concentration en composé conforme à l'invention dans la composition est au minimum de 0,1 nM et variera selon qu'on souhaite inhiber définitivement ou temporairement la croissance du bourgeon, la concentration étant en outre fonction de la nature de la plante à traiter. D'une manière générale, la concentration en composé dans la composition variera entre 0,1 nM et 10µM, préférentiellement entre 0,1 et 1000 nM, préférentiellement encore entre 1 et 100 nM.

De même, le nombre de jours de traitement peut varier en fonction de la plante, de son âge au moment du traitement, de l'effet définitif ou non souhaité, etc.

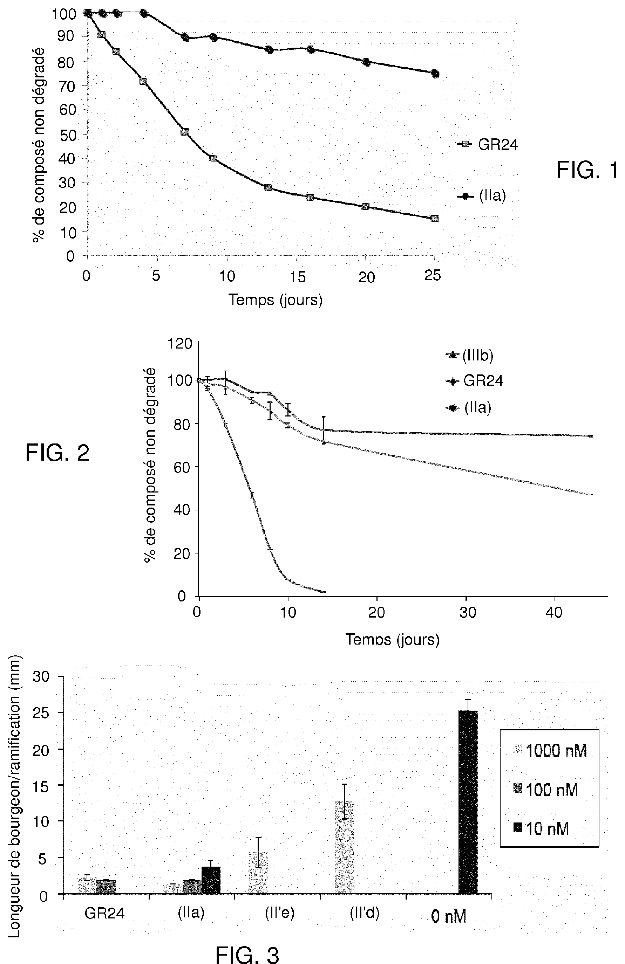

Les caractéristiques et avantages du procédé selon l'invention apparaîtront plus clairement à la lumière des exemples de mise en oeuvre ci-après, fournis à simple titre illustratif et nullement limitatifs de l'invention, avec l'appui des figures 1 à 17, dans lesquelles :
- la figure 1 est une courbe illustrant les résultats d'une analyse de stabilité chimique en solution aqueuse (50 µg/mL de composé dans méthanol/eau : 1/4, pH 6,7, 21 °C), pour le composé (IIa) conforme à l'invention et le composé comparatif GR24, le % de composé non dégradé dans la solution étant représenté en fonction du temps ;
- la figure 2 est une courbe illustrant les résultats d'une analyse de stabilité chimique en solution aqueuse (50 µg/mL de composé dans méthanol/eau : 1/4, pH 6,7, 21 °C), pour les composés respectifs (IIa) et (IIIb) conformes à l'invention et le composé comparatif GR24, le % de composé non dégradé dans la solution étant représenté en fonction du temps ;
- la figure 3 représente un graphe en barres montrant l'activité, exprimée en terme de longueur de bourgeon/ramification mesurée au noeud 3 8 jours après traitement au noeud 3, des composés conformes à l'invention (IIa), (II'd) et (II'e) et du composé comparatif GR24, à des doses comprises entre 10 nM et 1 µM, sur la répression de la ramification du mutant *rms1* du pois ;

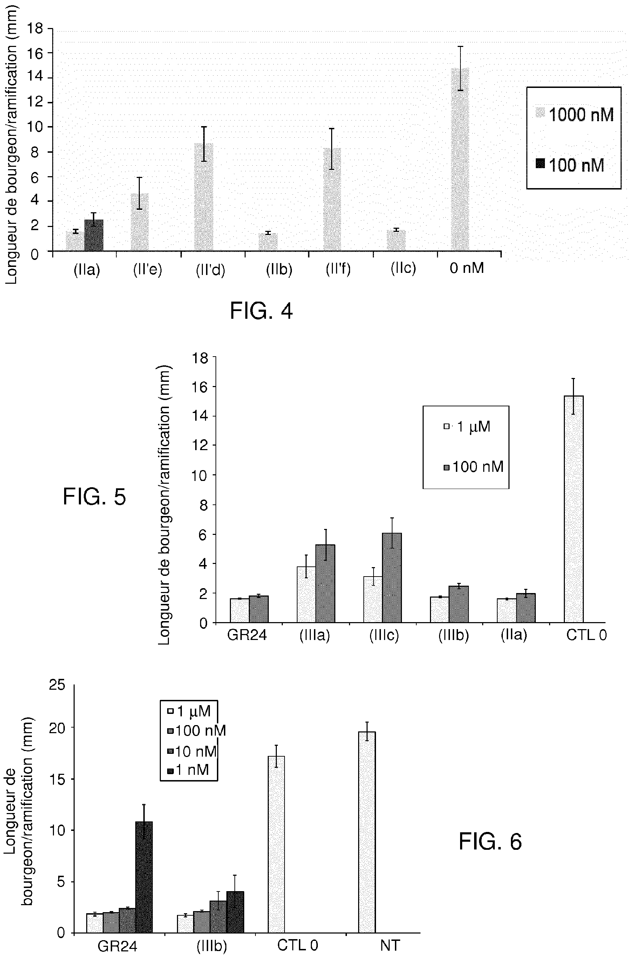

- la figure 4 représente un graphe en barres montrant l'activité, exprimée en terme de longueur de bourgeon/ramification mesurée au noeud 3 8 jours après traitement au noeud 3, des composés conformes à l'invention (IIa), (IIb), (IIc), (II'd), (II'e), (II'f), à des doses comprises entre 100 nM et 1 µM, sur la répression de la ramification du mutant *rms1* du pois ;
- la figure 5 représente un graphe en barres montrant l'activité, exprimée en terme de longueur de bourgeon/ramification mesurée au noeud 3 8 jours après traitement au noeud 3, des composés conformes à l'invention (IIa), (IIIa), (IIIb), (IIIc), et du composé comparatif GR24, à des doses respectives de 100 nM et 1 µM, sur la répression de la ramification du mutant *rms1* du pois ; un contrôle négatif « CTL 0 » représente un échantillon constitué du solvant seul ;
- la figure 6 représente un graphe en barres montrant l'activité, exprimée en terme de longueur de bourgeon/ramification mesurée au noeud 3 8 jours après traitement au noeud 3, du composé conforme à l'invention (IIIb) et du composé comparatif GR24, à des doses respectives de 1 nM, 10 nM, 100 nM et 1 µM, sur la répression de la ramification du mutant *rms1* du pois ; un contrôle négatif « CTL 0 » représente un échantillon constitué du solvant seul, et un contrôle négatif « NT » représente une plante non traitée ;

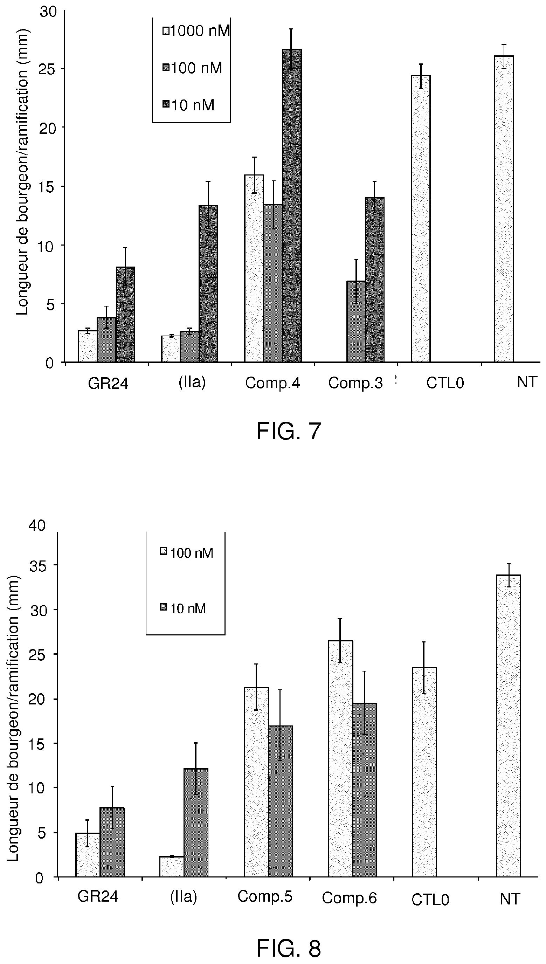

- la figure 7 représente un graphe en barres montrant l'activité, exprimée en terme de longueur de bourgeon/ramification mesurée au noeud 3 8 jours après traitement au noeud 3, du composé conforme à l'invention (IIa) et des composés comparatifs GR24, Comp.3 et Comp.4, à des doses respectives de 10 nM, 100 nM et 1 µM, sur la répression de la ramification du mutant *rms1* du pois ; un contrôle négatif « CTL 0 » représente un échantillon constitué du solvant seul, et un contrôle négatif « NT » représente une plante non traitée ;
- la figure 8 représente un graphe en barres montrant l'activité, exprimée en terme de longueur de bourgeon/ramification mesurée au noeud 3 8 jours après traitement au noeud 3, du composé conforme à l'invention (IIa) et des composés comparatifs GR24, Comp.5 et Comp.6, à des doses respectives de 10 nM et 100 nM, sur la répression de la ramification du mutant *rms1* du pois ; un contrôle négatif « CTL 0 » représente un échantillon constitué du solvant seul, et un contrôle négatif « NT » représente une plante non traitée ;

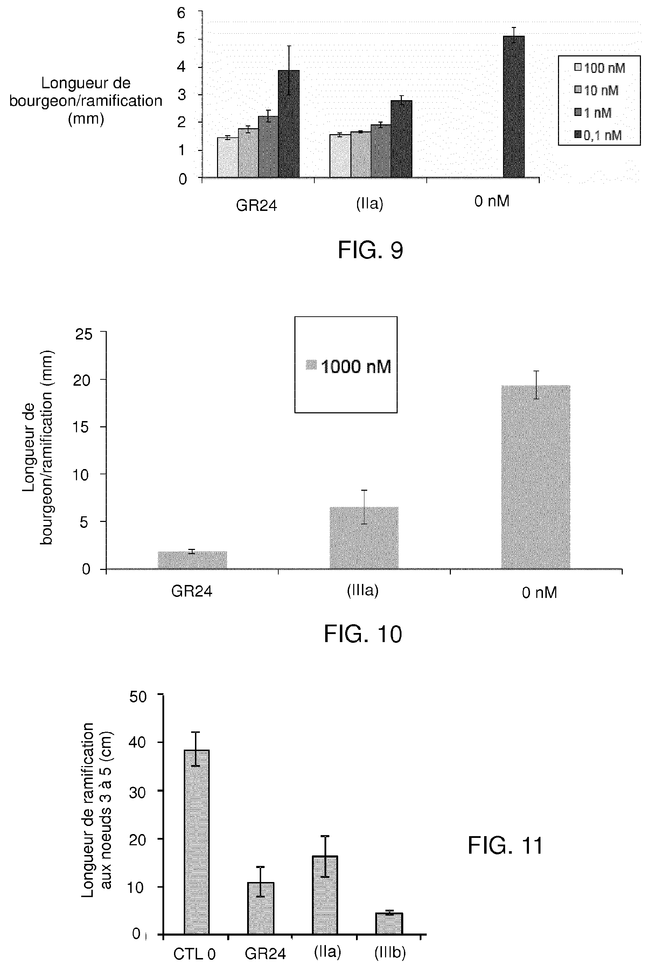

- la figure 9 représente un graphe en barres montrant l'activité, exprimée en terme de longueurs de bourgeon/ramification mesurée au noeud 4 8 jours après traitement au noeud 4, du composé (IIa) et du composé comparatif GR24 à des doses comprises entre 0,1 nM et 100 nM, sur la répression de la ramification du mutant *rms1* du pois ;
- la figure 10 représente un graphe en barres montrant l'activité, exprimée en terme de longueurs de bourgeon/ramification mesurée au noeud 3 8 jours après traitement au noeud 3, du composé conforme à l'invention (IIIa) et du composé comparatif GR24 à une dose de 1 µM, sur la répression de la ramification du mutant *rms1* du pois ;
- la figure 11 montre un graphe en barres montrant l'activité, exprimée en terme de longueur de ramification mesurée aux noeuds 3 à 5, 8 jours après le début d'une culture hydroponique de mutants *rms1* du pois, en présence des composés conformes à l'invention (IIa) et (IIIb) et du composé comparatif GR24 à une dose de 1 µM; un contrôle négatif « CTL 0 » représente un échantillon constitué du solvant seul ;

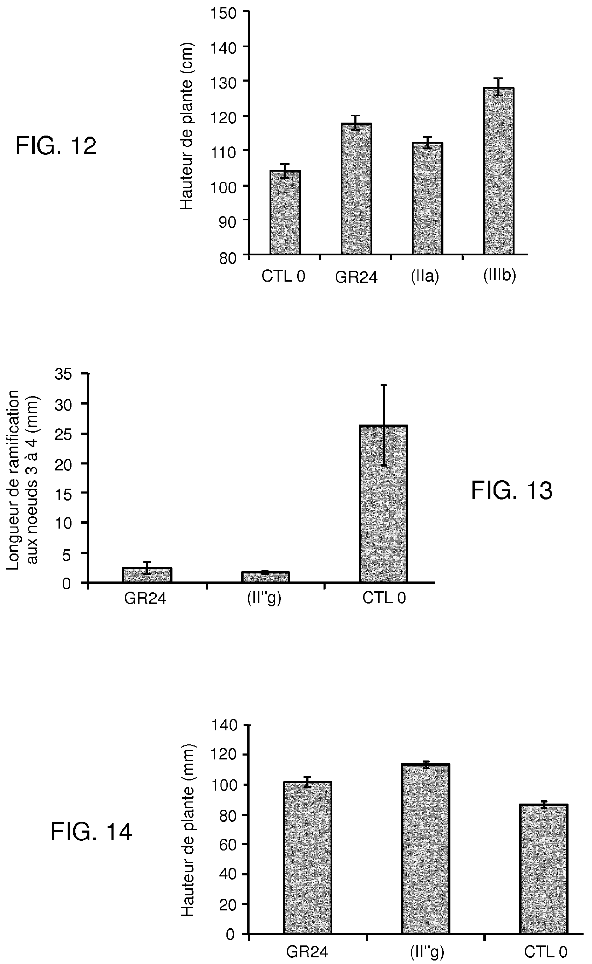

- la figure 12 montre un graphe en barres montrant l'activité, exprimée en terme de hauteur de plante, 8 jours après le début d'une culture hydroponique de mutants *rms1* du pois, en présence des composés conformes à l'invention (IIa) et (IIIb) et du composé comparatif GR24 à une dose de 1 µM; un contrôle négatif « CTL 0 » représente un échantillon constitué du solvant seul ;
- la figure 13 montre un graphe en barres montrant l'activité, exprimée en terme de longueur de ramification mesurée aux noeuds 3 à 4, 8 jours après le début d'une culture hydroponique de mutants *rms1* du pois, en présence du composé conforme à l'invention (II"g) et du composé comparatif GR24 à une dose de 3 µM; un contrôle négatif « CTL 0 » représente un échantillon constitué du solvant seul ;
- la figure 14 montre un graphe en barres montrant l'activité, exprimée en terme de hauteur de plante, 8 jours après le début d'une culture hydroponique de mutants *rms1* du pois, en présence du composé conforme à l'invention (II"g) et du composé comparatif GR24 à une dose de 3 µM ; un contrôle négatif « CTL 0 » représente un échantillon constitué du solvant seul ;

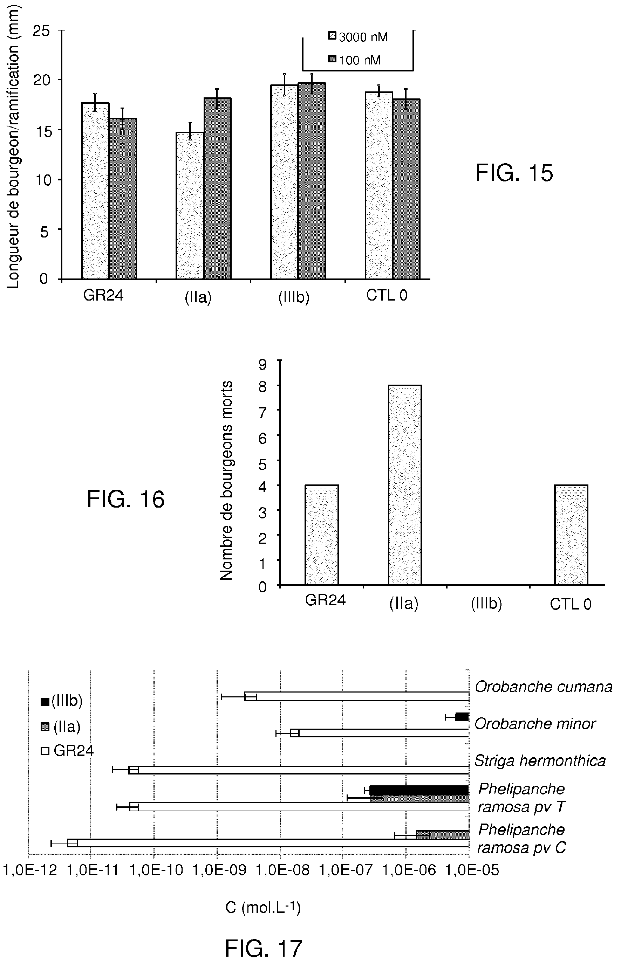

- la figure 15 représente un graphe en barres montrant l'activité, exprimée en terme de longueur de bourgeon/ramification mesurée au noeud 3 8 jours après traitement au noeud 3, des composés conformes à l'invention (IIa) et (IIIb), et du composé comparatif GR24, à des doses respectives de 100 nM et 3 µM, sur la répression de la ramification du mutant *rms4* du pois ; un contrôle négatif « CTL 0 » représente un échantillon constitué du solvant seul ;
- la figure 16 montre un graphe en barres représentant le nombre de bourgeons morts pour des groupes de 48 plantes mutantes *rms4* du pois, 8 jours après traitement au noeud 3 par les composés conformes à l'invention (IIa) et (IIIb), et le composé comparatif GR24, à des doses de 3 µM ; un contrôle négatif « CTL 0 » représente un échantillon constitué du solvant seul ;
- et la figure 17 est un graphe en barres montrant, pour les composés conformes à l'invention (IIa) et (IIIb) et pour le composé comparatif GR24, la concentration EC50 dans un essai de germination des plantes parasites : *Orobanche cumana, Orobanche minor, Striga hermonthica, Phelipanche ramosa (pathovar pv T* et *pv C).*

### EXEMPLE 1 - Synthèse d'exemples de composés selon l'invention et d'exemples de composés comparatifs

### 1/ Matériel et méthodes

Les spectres infrarouge ont été enregistrés en film sur une fenêtre en diamant. Les données sont données en cm⁻¹ (v, cm⁻¹).

Les spectres RMN ¹H et ¹³C ont été enregistrés sur des solutions dans CDCl₃, en utilisant le solvant protique CHCl₃ (δ_{H}=7,24 ppm) ou CDCl₃ (δ_{C}=77,23 ppm) comme référence interne et sont donnés en ppm.

Les spectres de masse ont été déterminés par ionisation par électro-nébulisation (ESI).

Toutes les réactions ont été suivies par chromatographie sur couche mince (CCM) sur des plaques d'aluminium de 0,2 mm pré-enduites de gel de silice, en utilisant la lumière UV et une solution éthanolique à 5 % en acide phosphomolybdique et la chaleur en tant qu'agent révélateur.

La Flash-chromatographie a été réalisée sur gel de silice 60, 40-63 µm (400-230 mesh), avec l'acétate d'éthyle (AcOEt) et l'heptane en tant qu'éluants.

Les réactifs et solvants disponibles commercialement ont été purifiés et séchés lorsque nécessaire par des méthodes classiques.

Le diméthylformamide DMF et le dichlorométhane CH₂Cl₂ ont été séchés par distillation sur hydrure de calcium, l'acétone par distillation sur CaSO₄ anhydre.

Sauf indication contraire, tous les autres réactifs ont été obtenus à partir des sources commerciales et utilisés sans autre purification.

### 2/ Composé (IIa)

Le composé répondant à la formule (IIa), le 5-((4-chlorophényl)thio)-3,4-diméthylfuran-2(5*H*)-one : 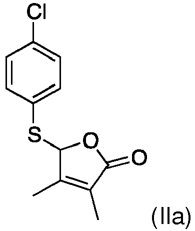 a été synthétisé à partir de 4-chlorothiophénol et de 5-chloro-3,4-diméthylfuran-2(5H)-one, selon le schéma réactionnel suivant : 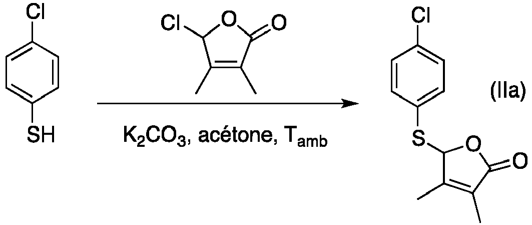 de la façon suivante.

A une solution de 4-chlorothiophénol commercial (400 mg, 2,76 mmol) dans de l'acétone anhydre (10 mL) avec du K₂CO₃ anhydre (459 mg, 3,31 mmol), placée à température ambiante sous argon, est additionnée une solution de 5-chloro-3,4-diméthylfuran-2(5*H*)-one (405 mg, 2,6 mmol) (préparé selon la méthode de Canevet et al., 1978) dans de l'acétone anhydre (5 ml). L'avancement de la réaction est suivi par CCM (SiO₂; Heptane/AcOEt 8:2).

Après 1 jour d'agitation, l'acétone est évaporée. Le brut est purifié par chromatographie sur silice (éluant Heptane/AcOEt 8:2) pour fournir le composé (IIa) sous forme d'un solide blanc (361 mg, 1,42 mmol, 51 %).
PF : 74,5-76,9°C.
RMN ¹H (300 MHz, CDCl₃) : δ 7,34 (dt, *J* = 8,7, 2,1 Hz, 2 H), 7,20 (dt, *J* = 8,7, 2,1 Hz, 2 H), 5,83-5,82 (m, 1 H), 1,96 (t, *J* = 1,0 Hz, 3 H), 1,64 (t, *J* = 1,0 Hz, 3 H).
RMN ¹³C (75 MHz, CDCl₃): δ 172,6 (C), 155,2 (C), 135,4 (C), 135,2 (2CH), 129,2 (2CH), 128,2 (C), 126,2 (C), 88,1 (CH), 12,6 (CH₃), 8,5 (CH₃).
IR νₘₐₓ (film) : 2924, 1758, 1673, 1476, 1093, 985 cm⁻¹.
HRMS (ESI): *m*/*z* calculée pour C₁₂H₁₂ClO₂S [M + H]⁺: 255,0247, trouvée : 255,0245.

### 3/ Composé (IIb)

Le composé répondant à la formule (IIb), le 5-((4-chlorophényl)thio)-3,4-diméthylfuran-2(5*H*)-one : 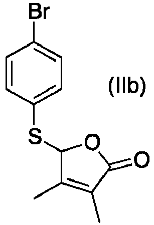 a été synthétisé à partir de 4-bromothiophénol et de 5-chloro-3,4-diméthylfuran-2(5H)-one, selon un schéma réactionnel similaire à celui indiqué ci-dessus pour le composé (IIa), de la façon suivante.

A une solution de 4-bromothiophénol commercial (283,6 mg, 1,5 mmol) dans de l'acétone anhydre (15 mL) avec du K₂CO₃ anhydre (337 mg, 3 mmol, 3 éq.), placée à température ambiante sous argon, est additionnée une solution de 5-chloro-3,4-diméthylfuran-2(5*H*)-one (263,8 mg, 1,8 mmol) dans de l'acétone anhydre (10 mL). L'avancement de la réaction est suivi par CCM (SiO₂ ; Heptane/AcOEt 8:2).

Après 8 jours d'agitation, l'acétone est évaporée et le résidu repris par du CH₂Cl₂ pour être filtré sur célite. Après évaporation, le solide marron résultant est purifié par chromatographie sur silice (introduction et élution au CH₂Cl₂, colonne de diamètre 2 cm et 45 cm de hauteur de silice) pour fournir le composé (IIb) sous forme d'un solide transparent (152,4 mg, 0,51 mmol, 34 % de rendement).
PF : 91 °C.
RMN ¹H (CDCl₃, 500 MHz) : *δ* (ppm) 1,71 (s large, 3H), 2,03 (s large, 3H), 5,89 (s large, 1 H), 7,38 (d, *J* = 8,2 Hz, 2H), 7,46 (d, *J* = 8,2 Hz, 2H).
RMN ¹³C (CDCl₃, 125.5 MHz) : *δ* (ppm) 8,7 (CH₃), 12,8 (CH₃), 88,2 (CH), 123,8 (C), 126,6 (C), 129,0 (C), 132,4 (2CH), 135,5 (2CH), 155,1 (C), 172,7 (C).
IR νₘₐₓ (film) : 1760, 1686, 1673 cm⁻¹.
MS (ES⁻) : *m*/*z* (%) 297,0 (99%, [M-H]⁻).
HRMS (ESI): *m*/*z* calculée pour C₁₂H₁₀O₂SBr [M-H]⁻ : 296,9585, trouvée : 296,9592.

### 4/ Composé (IIc)

Le composé répondant à la formule (IIc), le 3,4-diméthyl-5-((4-(trifluorométhyl)phényl)thio)furan-2(5H)-one : 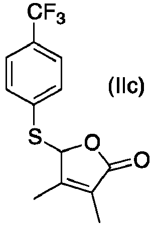 a été synthétisé à partir de 4-(trifluorométhyl)thiophénol et de 5-chloro-3,4-diméthylfuran-2(5H)-one, selon un schéma réactionnel similaire à celui indiqué ci-dessus pour le composé (IIa), de la façon suivante.

A une solution de 4-(trifluorométhyl)thiophénol commercial (267,3 mg, 1,5 mmol) dans de l'acétone anhydre (15 mL) avec du K₂CO₃ anhydre (337 mg, 3 mmol, 3 éq.), placée à température ambiante sous argon, est additionnée une solution de 5-chloro-3,4-diméthylfuran-2(5*H*)-one (263,8 mg, 1,8 mmol) dans de l'acétone anhydre (10 mL). L'avancement de la réaction est suivi par CCM (SiO₂; Heptane/AcOEt 8:2).

Après 8 jours d'agitation, l'acétone est évaporée et le résidu repris par du CH₂Cl₂ pour être filtré sur célite. Après évaporation, l'huile marron résultante est purifiée 2 fois par chromatographie sur silice (introduction et élution au CH₂Cl₂, colonne de diamètre 2 cm et 45 cm de hauteur de silice) pour fournir le composé (IIc) sous forme d'une huile jaune pâle transparente (112,8 mg, 0,39 mmol, 26% de rendement).
RMN ¹H (CDCl₃, 500 MHz) : δ (ppm) 1,75 (s large, 3H), 2,06 (s large, 3H), 5,99 (s large, 1 H), 7,57 (d, *J* = 8,6 Hz, 2H), 7,63 (d, *J* = 8,6 Hz, 2H).
RMN ¹³C (CDCl₃, 125,5 MHz) : δ (ppm) 8,7 (CH₃), 12,8 (CH₃), 88,0 (CH), 122,1 (C), 125,7 (CF₃), 126,0 (CH), 126,1 (CH), 126,8 (CH), 132,8 (2CH), 135,9 (C), 154,8 (C), 172,6 (C).
IR νₘₐₓ (film) : 1766, 1687, 1672 cm⁻¹.
MS (ES⁻) : *m*/*z* (%) 287,0 (100%, [M-H]⁻)
HRMS (ESI): *m*/*z* calculée pour C₁₃H₁₀O₂F₃S [M-H]⁻ : 287,0354, trouvée : 287,0342.

### 5/ Composé (II'd)

Le composé répondant à la formule (II'd), le 5-((4-chlorophénoxy)-3,4-diméthylfuran-2(5*H*)-one : 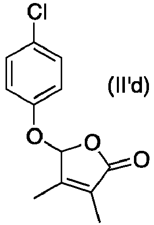 a été synthétisé à partir de 4-chlorophénol et de 5-chloro-3,4-diméthylfuran-2(5H)-one, selon un schéma réactionnel similaire à celui indiqué ci-dessus pour le composé (IIa), de la façon suivante.

A une solution de 4-chlorophénol commercial (0,847 g, 6,59 mmol) dans de l'acétone anhydre (50 mL) avec du K₂CO₃ anhydre (1,821 g, 13,13 mmol), placée à température ambiante sous argon, est additionnée une solution de 5-chloro-3,4-diméthylfuran-2(5*H*)-one (1,45 g, 9,89 mmol) (préparé selon la méthode de Canevet et al., 1978) dans de l'acétone anhydre (20 ml). L'avancement de la réaction est suivi par CCM (SiO₂; Heptane/AcOEt 8:2).

Après 2 jours d'agitation, l'acétone est évaporée. Le brut est purifié par chromatographie sur silice (éluant heptane/AcOEt 8:2) puis recristallisé (Hexane/AcOEt) pour fournir le composé (II'd) sous forme d'un solide blanc (793 mg, 3,33 mmol, 50%).
PF : 84-85 °C.
RMN ¹H (300 MHz, CDCl₃) : δ 7,27 (dt, *J* = 9,0, 3,2 Hz, 2 H), 7,06 (dt, *J* = 9,0, 3,0 Hz, 2 H), 6,00-5,99 (s large, 1 H), 2,05 (t, *J* = 1,0 Hz, 3 H), 1,87 (t, *J* = 1,0 Hz, 3 H).
RMN ¹³C (75 MHz, CDCl₃): δ 171,8 (C), 155,5 (C), 153,6 (C), 129,8 (2CH), 128,9 (C), 127,4 (C), 118,5 (2CH), 100,9 (CH), 11,8 (CH₃), 8,7 (CH₃).
IR νₘₐₓ (film) : 1773, 1694, 1490, 1227, 974 cm⁻¹.
MS (ES⁻) : *m*/*z* (%) 237,0 (100%, [M - H]⁻), 273,0 (90%, [M + Cl]⁻).
HRMS (ESI): *m*/*z* calculée pour C₁₂H₁₀ClO₃ [M - H]⁻ : 237,0318, trouvée : 237,0325.

### 6/ Composé (II'e)

Le composé répondant à la formule (II'e), le 5-((4-bromophénoxy)-3,4-diméthylfuran-2(5*H*)-one : 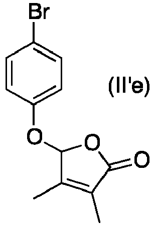 a été synthétisé à partir de 4-bromophénol et de 5-chloro-3,4-diméthylfuran-2(5H)-one, selon un schéma réactionnel similaire à celui indiqué ci-dessus pour le composé (IIa), de la façon suivante.

A une solution de 4-bromophénol commercial (259,5 mg, 1,5 mmol) dans de l'acétone anhydre (15 mL) avec du K₂CO₃ anhydre (337 mg, 3 mmol, 3 éq.), placée à température ambiante sous argon, est additionnée une solution de 5-chloro-3,4-diméthylfuran-2(5*H*)-one (245,0 mg, 1,67 mmol) dans de l'acétone anhydre (10 ml). L'avancement de la réaction est suivi par CCM (SiO₂ ; Heptane/AcOEt 8:2).

Après 17 jours d'agitation, l'acétone est évaporée et le résidu repris par du CH₂Cl₂ pour être filtré sur célite. Après évaporation, le solide marron résultant est purifié par chromatographie sur silice (introduction et élution au CH₂Cl₂, colonne de diamètre 2 cm et 45 cm de hauteur de silice) pour fournir le composé (II'e) sous forme d'un solide transparent (241,7 mg, 0,85 mmol, 57 % de rendement).
PF: 94°C.
RMN ¹H (CDCl₃, 500 MHz) : δ (ppm) 1,89 (s large, 3H), 2,07 (s large, 3H), 6,02 (s large, 1 H), 7,03 (d, *J* = 9,0 Hz, 2H), 7,43 (d, *J* = 9,0 Hz, 2H).
RMN ¹³C (CDCl₃, 125,5 MHz) : δ (ppm) 8,7 (CH₃), 11,7 (CH₃), 100,8 (CH), 116,3 (C), 118,9 (2CH), 127,3 (C), 132,8 (2CH), 153,5 (C), 156,0 (C), 171,7 (C).
IR (film) : ν (cm⁻¹) 1772, 1693, 1582.
MS (ES⁻) : *m*/*z* (%) 281,0 (99%, [M-H]⁻).
HRMS : *m*/*z* (280,9809, [M-H]⁻) ; calculée pour C₁₂H₁₀O₃Br : 280,9813.

### 7/ Composé (II'f)

Le composé répondant à la formule (II'f), le 3,4-diméthyl-5-(4-(trifluorométhyl)phénoxy)furan-2(5H)-one : 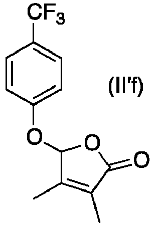 a été synthétisé à partir de 4-(trifluorométhyl)phénol et de 5-chloro-3,4-diméthylfuran-2(5H)-one, selon un schéma réactionnel similaire à celui indiqué ci-dessus pour le composé (IIa), de la façon suivante.

A une solution de 4-(trifluorométhyl)phénol commercial (243,2 mg, 1,5 mmol) dans de l'acétone anhydre (15 mL) avec du K₂CO₃ anhydre (337 mg, 3 mmol, 3 éq.), placée à température ambiante sous argon, est additionnée une solution de 5-chloro-3,4-diméthylfuran-2(5*H*)-one (263,4 mg, 1,8 mmol) dans de l'acétone anhydre (10 ml). L'avancement de la réaction est suivi par CCM (SiO₂ ; Heptane/AcOEt 8:2).

Après 8 jours d'agitation, l'acétone est évaporée et le résidu repris par du CH₂Cl₂ pour être filtré sur célite. Après évaporation, le solide marron résultant est purifié par chromatographie sur silice (introduction et élution au CH₂Cl₂, colonne de diamètre 2 cm et 45 cm de hauteur de silice) pour fournir le composé (II'f) (132,2 mg, 32 %) sous forme d'un solide transparent.
PF: 97°C.
RMN ¹H (CDCl₃, 500 MHz) : δ (ppm) 1,91 (s large, 3H), 2,09 (s large, 3H), 6,12 (s large, 1 H), 7,22 (d, *J* = 8,6 Hz, 2H), 7,61 (d, *J* = 8,6 Hz, 2H).
RMN ¹³C (CDCl₃, 125,5 MHz) : δ (ppm) 8,7 (CH₃), 11,7 (CH₃), 100,1 (CH), 116,9 (2CH), 122,4 (C), 126,0 (CF₃), 127,3 (2CH), 127,4 (C), 153,4 (C), 159,2 (C), 171,6 (C).
IR (film) : ν (cm⁻¹) 1768, 1697, 1614.
MS (ES⁻): *m*/*z* (%) 271,1 (100%, [M-H]⁻).
HRMS : *m*/*z* (271,0579, [M-H]⁻) ; calculée pour C₁₃H₁₀O₃F₃: 271,0582.

### 8/ Composé (II"g)

Le composé répondant à la formule (II"g), le 7-[3,4-diméthylfuran-2(5*H*)-one)]oxy coumarine : 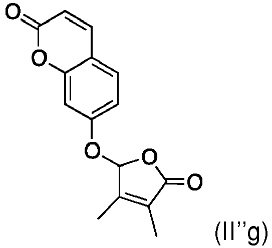 a été synthétisé à partir de la 7-hydroxycoumarine et du 5-chloro-3,4-diméthylfuran-2(5H)-one, selon le schéma réactionnel suivant : 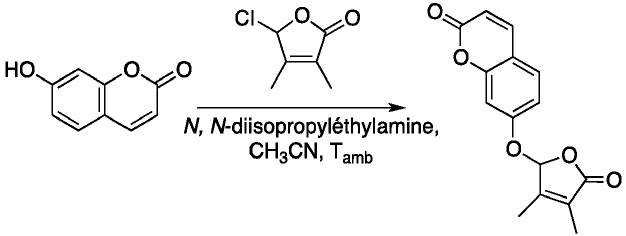 de la façon suivante.

A une solution de 5-chloro-3,4-diméthylfuran-2(5*H*)-one (352 mg, 2 mmol) (préparé selon la méthode de Cavenet et al. (1978)) dans de l'acétonitrile anhydre (10 mL) placée à température ambiante sous argon, est additionnée de la 7-hydroxycoumarine solide commerciale ou facilement accessible selon la méthode de Timonen et al. (2011) (CAS RN : 93-35-6, 300 mg, 1,85 mmol). De la N,N-Diisopropyléthylamine est alors ajoutée (697 µL, 4 mmol). L'avancement de la réaction est suivi par CCM (SiO₂; Heptane/AcOEt 1:1).

Après 12 h d'agitation, l'acétonitrile est évaporée. Le brut est purifié par chromatographie sur silice (éluant Heptane/AcOEt 6:4) pour fournir le composé (II"g) sous forme d'un solide blanc (423 mg, 1,55 mmol, rendement : 84 %).
PF : 176,2°C.
RMN ¹H (300 MHz, CDCl₃) : δ 1,85 (t, J = 1,2 Hz, 3H), 2,04 (t, J = 0,9 Hz, 3H), 6,08 (s, 1 H), 6,24-6,27 (d, J = 9,5 Hz, 1 H), 6,98-7,01 (m, 2H), 7,37-7,39 (d, J = 8,1 Hz, 1 H), 7,58-7,61 (d, J = 9,6 Hz, 1 H).
RMN ¹³C (75 MHz, CDCl₃): δ 8,7(CH₃), 11,7(CH₃), 99,9(CH), 104,6(CH), 113,5(CH), 114,8(CH), 114,9(CH), 127,5(C), 129,3(CH), 143,1 (CH), 153,3(C), 155,5(C), 159,5(C), 160,7(C), 171,4(C).
IR νₘₐₓ (film) : 661, 750, 834, 886, 975, 1052, 1088, 1131, 1162, 1195, 1236, 1285, 1318, 1361, 1387, 1505, 1565, 1615, 1624, 1689, 1745, 1781, 3081 cm⁻¹.
HRMS (ESI): *m*/*z* calculée pour C₁₅H₁₃O₅ [M + H]⁺ : 273,0718, trouvée : 273,0753.

### 9/ Composé (IIIa)

Le composé répondant à la formule (IIIa), le 3-((3,4-diméthyl-5-oxo-2,5-dihydrofuran-2-yl)oxy)-2-méthylacrylaldéhyde : 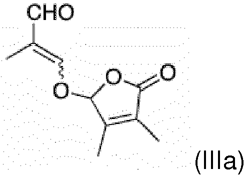 a été synthétisé à partir du sel de sodium du méthylmalondialdéhyde et de 5-chloro-3,4-diméthylfuran-2(5H)-one, selon le schéma réactionnel ci-dessous :

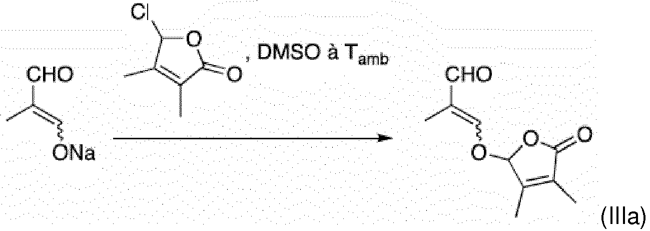

Pour cela, le sel de sodium du méthylmalondialdéhyde (obtenu conformément à la publication de Nair et al., 1981) (244 mg, 2,6 mmol), le 5-chloro-3,4-diméthylfurane-2(5H)-one (165 mg, 1,13 mmol) et du diméthylsulfoxyde (2 mL) sont mélangés et agités à température ambiante pendant 14 h. L'achèvement de la réaction est contrôlé par CCM (heptane/EtOAc, 8/2 v/v). De l'eau est ensuite ajoutée au mélange jusqu'à entière dissolution des sels, puis la phase aqueuse est extraite 3 fois au dichlorométhane. Les phases organiques réunies sont séchées avec du Na₂SO₄, filtrées et évaporées sous pression réduite jusqu'à obtenir un résidu sec.

Le produit brut est purifié par chromatographie sur gel de silice avec un gradient linéaire de 0-50 % v/v d'AcOEt dans l'heptane, pour obtenir le composé souhaité (IIIa) (202 mg, 1,03 mmol, rendement 91 %) sous forme de solide blanc.
Rf = 0,13 (heptane/EtOAc, 8/2 v/v)
RMN ¹H (500 MHz, CDCl₃) : 8 : 9,36 (s, 1H), 7,18 (s, 1H), 6,01 (s, 1H), 2,08 (s, 3H), 1,94 (s, 3H), 1,73 (s, 3H).
RMN ¹³C (125 MHz, CDCl₃) δ : 191,5 (CH), 170,9 (C), 161,4 (CH), 152,6 (C), 128,2 (C) 123,3 (C), 101,8 (CH), 11,4 (CH₃), 8,8 (CH₃), 6,6 (CH₃)
IR νₘₐₓ (film) : 1771, 1684, 1652, 1168, 978 cm⁻¹.
MS (ESI) : *m*/*z* = 197 (MH⁺, 100%).
HRMS (ESI, mode positif) : Calculée pour C₁₀H₁₃O₄ [M + H⁺] : 197,0814. Trouvée : 197,0809.

### 10/ Composé (IIIb)

Le composé répondant à la formule (IIIb), le 5-((3,4-diméthyl-5-oxo-2,5-dihydrofuran-2-yl)oxy)-4-méthylpenta-2,4-dienoate : 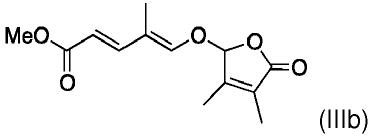 est synthétisé à partir du composé (IIIa) ci-dessus, selon le schéma réactionnel ci-après :
Le composé (IIIa) (100 mg, 0,5 mmol) est dissous dans du toluène sec (10 mL). Du méthyl(triphénylphosphoranylidène)acétate (203 mg, 0,61 mmol) disponible commercialement est ajouté et le mélange résultant est agité sous reflux pendant 12 h. L'achèvement de la réaction est contrôlé par CCM (heptane/EtOAc, 7:3, v/v). Le mélange réactionnel est ensuite concentré sous pression réduite et le résidu est purifié par chromatographie sur une colonne de gel de silice avec un gradient linéaire d'EtOAc (0-20 %) dans l'heptane en tant que phase mobile, pour obtenir le diénoate souhaité (IIIb) sous forme d'une poudre blanche (77 mg, 305 *µ*mol, rendement = 80 % après élimination de l'aldéhyde de départ (IIIa) n'ayant pas réagi).
RMN ¹H (500 MHz, CDCl₃) δ : 7,23 (d, *J* = 15,4 Hz, 1 H), 6,69 (s, 1 H), 5,85 (s, 1 H), 5,79 (d, *J* = 15,4 Hz, 1 H), 3,72 (s, 3H), 1,99 (s, 3H), 1,87 (s, 3H), 1,73 (s, 3H).
RMN ¹³C (125 MHz, CDCl₃) δ : 171,2, 167,7, 153,0, 148,3, 144,9, 127,8, 117,4, 115,1, 101,6, 51,5, 11,4, 9,4, 8,6.
IR νₘₐₓ (film, cm⁻¹) : 1767, 1718, 1639, 1317, 1154, 979.
HRMS (ESI, mode positif) : *m*/*z* = 253,1076 [M+H]⁺, calculée pour C₁₃H₁₇O₅ = 253,1031.

### 11/ Composé (IIIc)

Le composé répondant à la formule (IIIc), le (E)-3,4-diméthyl-5-((2-méthylbuta-1,3-dien-1-yl)oxy)furan-2(5H)-one : 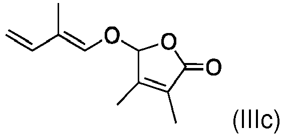 est synthétisé à partir du composé (IIIa) ci-dessus, selon le schéma réactionnel ci-après :
Du bromure de méthyl triphénylphosphonium (1,25 g, 3,5 mmol) est dissous dans du THF sec (4 mL) et refroidi à -50 °C. Du butyllithium (2,25 mL, 3,6 mmol, 1,6 M dans l'hexane) est ajouté goutte à goutte et le mélange résultant est réchauffé jusqu'à -10 °C pendant 45 min, et refroidi à -78 °C. Une solution de (IIIa) (400 mg, 2,04 mmol) dans du THF (4 mL) est ajoutée goutte à goutte et le mélange est agité pendant 2h30 à -50 °C. Le mélange réactionnel est ensuite laissé réchauffer jusqu'à -20 °C avant d'être versé dans un mélange de CH₂Cl₂ et de tampon phosphate (pH 7) (v/v; 1/1). Le mélange est extrait 3 fois au CH₂Cl₂, les phases organiques rassemblées sont lavées à l'eau, séchée sous pression réduite et le résidu résultant est purifié par chromatographie sur colonne de gel de silice avec un gradient linéaire d'EtOAc (0-50 %) dans l'heptane en tant que phase mobile, pour obtenir le diène souhaité (IIIc) sous forme d'une huile jaune pâle (150 mg, 773 *µ*mol, rendement = 38 %).
RMN ¹H (500 MHz, CDCl₃) δ : 6,34 (s, 1 H), 6,69 (s, 1 H), 6,22 (dd, *J* = 17,2, 10,7 Hz, 1 H), 5,77 (s, 1 H), 5,08 (d, *J* = 17,2 Hz, 1 H), 4,94 (d, *J* = 10,7 Hz, 1 H), 1,98 (s, 3H), 1,85 (s, 3H), 1,71 (s, 3H).
RMN ¹³C (125 MHz, CDCl₃) δ : 171,6, 154,2, 142,4, 135,5, 127,4, 119,2, 111,0, 101,8, 11,4, 9,04, 8,4.
IR νₘₐₓ (film, cm⁻¹): 1766, 1150, 960.
HRMS (ESI, mode positif): *m*/*z* = 195,1020 [M+H]⁺, calculée pour C₁₁H₁₅O₃ = 195,1021.

### 12/ Composé comparatif 1 - GR24

Le GR24 a été préparé selon la méthode connue en elle-même décrite dans Mangnus et al., 1992.

### 13/ Composé comparatif 2 - GR5

Le GR5 a été synthétisé selon la méthode connue en elle-même décrite dans Johnson et al., 1981.

### 14/ Composé comparatif 3 - Comp.3

Le composé comparatif Comp.3, de formule générale : 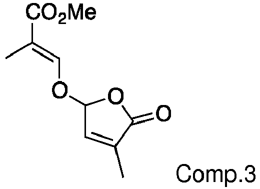 qui diffère des composés selon l'invention, en particulier des composés répondant à la formule (III), en ce que R¹ et R² représentent tous deux un atome d'hydrogène, est préparé selon le protocole décrit dans le document de brevet WO 2010/137662.

### 15/ Composés comparatifs 4 à 6 - Comp.4, Comp.5, Comp.6

Les composés comparatifs Comp.4, Comp.5, Comp.6, de formules générales respectives : 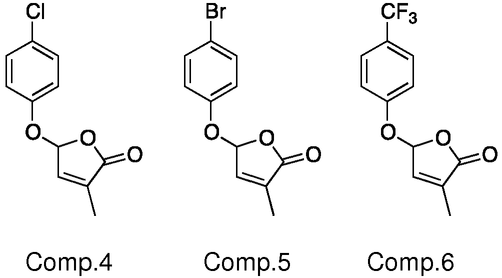 qui diffèrent des composés selon l'invention, en particulier des composés répondant à la formule (II'), en ce que R¹ et R² représentent tous deux un atome d'hydrogène, sont préparés selon les protocoles décrits dans le document de brevet WO 2012/043813 et dans la publication de Fukui et al. (2011)..

### EXEMPLE 2 - Etudes de stabilité en milieu aqueux

### 1/ Composé (IIa)

La stabilité en milieu aqueux du composé (IIa) selon l'invention et celle du GR24 en tant que composé comparatif, a été évaluée de la façon suivante.

Chaque composé a été dilué dans de l'acétone (1 mL), puis 50 µL de chaque solution ont été dilués avec du méthanol (175 µL) et de l'eau (750 µL) pour obtenir une concentration en composé de 50 µg/mL.

La solution aqueuse ainsi obtenue a été incubée à 21 °C dans des fioles HPLC.

De l'indanol (25 µL d'une solution à 1 mg/mL dans l'acétone) a été ajouté à chaque solution pour servir d'étalon interne.

La dégradation de chaque composé dans le temps a été suivie par analyse d'échantillons prélevés de la solution à différents intervalles de temps, par Chromatographie Liquide Ultra Performance (UPLC) au moyen d'une colonne Acquity UPLC HSS C₁₈ (1,8 µm, 2,1 × 50 mm), éluée tout d'abord par une solution d'acétonitrile à 5 % dans l'eau contenant 0,1 % d'acide formique, pendant 0,5 min, puis par un gradient de 5 % à 100 % d'acétonitrile dans l'eau contenant 0,1 % d'acide formique, pendant 6,5 min, et par 100 % d'acétonitrile contenant 0,1 % d'acide formique pendant 3 min. La colonne a été maintenue à une température de 40°C, avec un flux de 0,6 mL/min.

Les composés élués de la colonne ont été détectés avec un détecteur à barrettes de photodiodes.

La quantité relative de composé non dégradé dans la solution a été déterminée par comparaison avec l'étalon interne.

Les résultats obtenus sont illustrés sur la courbe de la Figure 1, en % de composé non dégradé dans la solution en fonction du temps d'incubation.

On y observe clairement que le composé (IIa) présente une stabilité chimique en solution aqueuse très élevée, et bien supérieure à celle du GR24.

### 2/ Composés (IIa) et (IIIb)

Dans une deuxième expérience, la stabilité en milieu aqueux des composés (IIa) et (IIIb) selon l'invention, et celle du GR24 en tant que composé comparatif, a été évaluée selon le protocole décrit ci-avant.

Les résultats obtenus sont illustrés sur la Figure 2, en % de composé non dégradé dans la solution, en fonction du temps d'incubation.

Les résultats obtenus, y compris sur des durées d'incubation plus longues, confirment que le composé (IIa) présente une stabilité chimique en solution aqueuse très élevée, bien supérieure à celle du GR24. La stabilité en milieu aqueux du composé (IIIb) est en outre meilleure encore que celle du composé (IIa).

### EXEMPLE 3 - Etude de cytotoxicité in vitro

La cytotoxicité du composé (IIa) selon l'invention et celle du GR24 et du GR5 en tant que composés comparatifs, a été évaluée *in vitro,* à des concentrations de 10⁻⁴ M et 10⁻⁵ M, sur des cellules MRC5 dans du DMSO, de la façon suivante.

Des cellules MRC5 (fibroblastes de poumon humain) ont été cultivées dans du milieu Eagle modifié de Dulbecco (DMEM) supplémenté par : 25 mM de glucose, 10 %(v/v) de sérum de veau foetal, 100 Ul de pénicilline, 100 µg/ml de streptomycine et 1,5 µg/ml de fungizone, et conservées sous 5 % de CO₂ à 37 °C.

Des plaques 96 puits ont été ensemencées par 2100 cellules MRC5 par puits dans 200 µl de milieu. Après 24 heures, chacun des composés dissout dans du DMSO a été ajouté pendant 72 heures, à une concentration finale de 10⁻⁴ M ou 10⁻⁵ M dans un volume de DMSO fixé. Les contrôles ont reçu un volume égal de DMSO.

Le nombre de cellules viable a été mesuré à 490 nm avec le réactif MTS (Promega), et pour chaque composé la concentration causant 50 % d'inhibition de la croissance cellulaire IC₅₀ a été calculée.

Les résultats, en terme de pourcentage d'inhibition de la croissance cellulaire (± erreur standard), sont montrés dans le Tableau 1 ci-dessous.

**Tableau 1 - Pourcentage d'inhibition de la croissance de cellules MRC5 du composé (IIa) conforme à l'invention et des composés comparatifs GR5 et GR24 aux concentrations de 10⁻⁴ M et 10⁻⁵ M**

| | Pourcentage d'inhibition de la croissance de cellules MRC5 | |
|---|---|---|
| | 10⁻⁴ M | 10⁻⁵ M |
| GR5 | 66 ± 1 | 0 ± 2 |
| GR24 | 97 ± 1 | 0 ± 11 |
| (IIa) | 11 ± 5 | 7 ± 4 |

Les résultats ci-dessus montrent clairement qu'à la concentration de 10⁻⁴ M, le composé conforme à l'invention (IIa) présente une cytotoxicité nettement moindre que celle des composés comparatifs GR5 et GR24.

### EXEMPLE 4 - Etude de l'activité biologique sur l'inhibition de la ramification chez le pois

Il a été utilisé à cet effet des mutants *ramosus* (*rms1*) hyper-ramifiés du pois (*Pisum sativum L.*), connus pour présenter un nombre de ramifications très supérieur au nombre de ramifications chez le pois sauvage et notamment à tous les noeuds de la plante. D'une manière générale, chez le pois, les deux premières écailles sont considérées comme les deux premiers noeuds, le noeud cotylédonaire étant le noeud 0.

Le mutant *rms1* est un mutant de biosynthèse du signal dit SMS, réprimant la ramification de la plante.

Il a été conduit des tests d'activité sur l'inhibition de la ramification, de la façon suivante.

Les mutants de pois *rms1* (*ccd8*) décrits dans Beveridge et al. 1997, déficients en strigolactones, ont été utilisés pour le test (lignée M3T884 issue de la variété Térèse).

### 1/ Expérience 1

L'activité des composés (IIa), (II'd) et (II'e) conformes à l'invention et du composé comparatif GR24 a été évaluée à des doses respectives de 10 nM, 100 nM et 1 µM, par traitement au noeud 3.

Pour cela, il a été utilisé des solutions contenant chaque composé à tester dans 1 % acétone, 4 % polyéthylène glycol 1450, 50 % éthanol.

Il a été semé 24 plants par traitement. 8 jours après semis, le traitement a été effectué sur le bourgeon axillaire au noeud 3, par application de 10 µl de chaque solution à tester directement sur le bourgeon, au moyen d'une micropipette. Les excroissances latérales aux noeuds 1 et 2 ont été ôtées pour encourager la croissance des bourgeons axillaires aux noeuds supérieurs.

Le démarrage des bourgeons axillaires au noeud 3 a été mesuré 8 jours après le traitement au moyen d'un pied à coulisse digital.

Les résultats, exprimés en longueur de bourgeon/ramification 8 jours après traitement par chaque composé, pour chaque concentration testée, sont illustrés sur la Figure 3. Le témoin sans traitement (0 nM) est également représenté sur cette figure.

On y observe que le composé (IIa) présente aux plus fortes concentrations une activité d'inhibition de la croissance des bourgeons supérieure à celle du composé comparatif GR24. Les composés (II'd) et (II'e) présentent également une activité d'inhibition de la croissance des bourgeons au noeud 3, à la concentration de 1 µM.

### 2/ Expérience 2

L'activité des composés (IIa), (IIb), (IIc), (II'd), (II'e) et (II'f) conformes à l'invention a été évaluée à des doses respectives de 100 nM et 1 µM, par traitement au noeud 3.

Pour cela, il a été utilisé des solutions contenant chaque composé à tester dans 1 % acétone, 4 % polyéthylène glycol 1450, 50 % éthanol.

Il a été semé 24 plants par traitement. 8 jours après semis, le traitement a été effectué sur le bourgeon axillaire au noeud 3, par application de 10 µl de chaque solution à tester directement sur le bourgeon, au moyen d'une micropipette. Les excroissances latérales aux noeuds 1 et 2 ont été ôtées pour encourager la croissance des bourgeons axillaires aux noeuds supérieurs.

Le démarrage des bourgeons axillaires au noeud 3 a été mesuré 8 jours après le traitement au moyen d'un pied à coulisse digital.

Les résultats, exprimés en longueur de bourgeon/ramification 8 jours après traitement par chaque composé, pour chaque concentration testée, sont illustrés sur la Figure 4. Le témoin sans traitement (0 nM) est également représenté sur cette figure.

On y observe que l'ensemble des composés conformes à l'invention présente une activité d'inhibition de la croissance des bourgeons au noeud 3. Les composés comportant un atome de soufre dans la molécule (composés (IIa), (IIb) et (IIc)) s'avèrent être les plus actifs.

### 3/ Expérience 3

L'activité des composés (IIa), (IIIa), (IIIb) et (IIIc) conformes à l'invention, ainsi que du GR24 en tant que composé comparatif, a été évaluée à des doses respectives de 100 nM et 1 µM, par traitement au noeud 3, selon le protocole décrit ci-dessus en référence à l'Expérience 2.

En tant que contrôle négatif, dit « CTL 0 », un échantillon constitué uniquement du solvant, et sans composé actif, a également été testé.

Les résultats, exprimés en longueur de bourgeon/ramification 8 jours après traitement par chaque composé, pour chaque concentration testée, sont illustrés sur la Figure 5.

On observe que les composés (IIa) et (IIIb) conformes à l'invention présentent une activité similaire à celle du composé comparatif GR24. Les composés (IIIa) et (IIIc), bien que légèrement moins actifs, présentent cependant une activité élevée en terme d'inhibition de la croissance des bourgeons.

### 4/ Expérience 4

Dans cette expérience, l'activité du composé (IIIa) conforme à l'invention, ainsi que du GR24 en tant que composé comparatif, a été évaluée à des doses respectives de 1 nM, 10 nM, 100 nM et 1 µM, par traitement au noeud 3, selon le protocole décrit ci-dessus en référence à l'Expérience 2, pour des doses de 10 nM, 100 nM et 1 µM de composés.

En tant que contrôle négatif, un échantillon constitué uniquement du solvant, et sans composé actif (« CTL 0 »), a également été testé. Un témoin non traité (« NT ») a en outre été réalisé.

Les résultats, exprimés en longueur de bourgeon/ramification 8 jours après traitement par chaque composé, pour chaque concentration testée, sont illustrés sur la Figure 6.

On y observe que le composé (IIIb) conforme à l'invention présente une activité supérieure à celle du composé comparatif GR24, en particulier aux doses les plus faibles.

### 5/ Expérience 5

Un test d'évaluation, dose-réponse, de l'activité des composés par dépôt au noeud 3 a été réalisé selon le protocole décrit ci-dessus en référence à l'Expérience 2, pour le composé conforme à l'invention (IIa) et pour les composés comparatifs GR24, Comp.3 et Comp.4. Des concentrations de 10 nM, 100 nM et 1 µM ont été testées.

En tant que contrôle négatif, un échantillon constitué uniquement du solvant, et sans composé actif (« CTL 0 »), a également été testé. Un témoin non traité (« NT ») a en outre été réalisé.

Les résultats obtenus (données moyennes sur 24 plantes) sont montrés sur la Figure 7. On y observe que le composé conforme à l'invention (IIa) présente une activité supérieure aux composés comparatifs proposés par l'art antérieur Comp.3 et Comp.4, et ce à toutes les doses testées.

### 6/ Expérience 6

Un test d'évaluation, dose-réponse, de l'activité des composés par dépôt au noeud 3 a été réalisé selon le protocole décrit ci-dessus en référence à l'Expérience 2, pour le composé conforme à l'invention (IIa) et pour les composés comparatifs GR24, Comp.5 et Comp.6. Des concentrations de 10 nM et 100 nM ont été testées.

En tant que contrôle négatif, un échantillon constitué uniquement du solvant, et sans composé actif (« CTL 0 »), a également été testé. Un témoin non traité (« NT ») a en outre été réalisé.

Les résultats obtenus (données moyennes sur 24 plantes) sont montrés sur la Figure 8. On y observe que le composé conforme à l'invention (IIa) présente une activité supérieure aux composés comparatifs proposés par l'art antérieur Comp.5 et Comp.6, et ce à toutes les doses testées.

### 7/ Expérience 7

L'activité du composé (IIa) conforme à l'invention sur la croissance des bourgeons au noeud 4 a été évaluée à des doses respectivement de 0,1 nM, 1 nM, 10 nM et 100 nM, par traitement au noeud 4. Le composé GR24 a été testé aux mêmes concentrations en tant que composé comparatif.

Pour cela, il a été utilisé des solutions contenant chaque composé à tester dans 1 % acétone, 4 % polyéthylène glycol 1450, 50 % éthanol.

Il a été semé 24 plants par traitement. 8 jours après semis, le traitement a été effectué sur le bourgeon axillaire au noeud 4, par application de 10 µl de chaque solution à tester directement sur le bourgeon, au moyen d'une micropipette. Les excroissances latérales aux noeuds 1 et 2 ont été ôtées pour encourager la croissance des bourgeons axillaires aux noeuds supérieurs.

Le démarrage des bourgeons axillaires au noeud 4 a été mesuré 8 jours après le traitement au moyen d'un pied à coulisse digital.

Les résultats, exprimés en longueur de bourgeon/ramification 8 jours après traitement par chaque composé, pour chaque concentration testée, sont illustrés sur la Figure 9. Le témoin sans traitement (0 nM) est également représenté sur cette figure.

On y observe le composé (IIa) conforme à l'invention présente, après traitement au noeud 4, une activité d'inhibition de la croissance des bourgeons au noeud 4 qui est équivalente à celle du GR24 aux concentrations les plus fortes, et légèrement meilleure à la concentration la plus basse de 0,1 nM.

### 8/ Expérience 8

L'activité du composé (IIIa) conforme à l'invention sur la croissance des bourgeons au noeud 3 a été évaluée à une dose 1000 nM par traitement au noeud 3. Le composé GR24 a été testé à la même concentration en tant que composé comparatif.

Pour cela, il a été utilisé des solutions contenant chaque composé à tester dans 1 % acétone, 4 % polyéthylène glycol 1450, 50 % éthanol.

Il a été semé 24 plants par traitement. 8 jours après semis, le traitement a été effectué sur le bourgeon axillaire au noeud 3, par application de 10 µl de chaque solution à tester directement sur le bourgeon, au moyen d'une micropipette. Les excroissances latérales aux noeuds 1 et 2 ont été ôtées pour encourager la croissance des bourgeons axillaires aux noeuds supérieurs.

Le démarrage des bourgeons axillaires au noeud 3 a été mesuré 8 jours après le traitement au moyen d'un pied à coulisse digital.

Les résultats, exprimés en longueur de bourgeon/ramification 8 jours après traitement par chaque composé, pour chaque concentration testée, sont illustrés sur la Figure 10. Le témoin sans traitement (0 nM) est également représenté sur cette figure.

On y observe le composé (IIIa) conforme à l'invention présente, après traitement au noeud 3, une activité d'inhibition de la croissance des bourgeons au noeud 3 qui, bien qu'inférieure à celle du GR24, est tout à fait importante.

### EXEMPLE 5 - Test de culture hydroponique chez le pois

Il a été utilisé pour cet exemple des mutants *ramosus* (*rms1*) hyper-ramifiés du pois (*Pisum sativum L.*)*.*

Plus particulièrement, les plantes mutantes hyper-ramifiées *rms1* (*rms1-10*) ont été utilisées. La solution nutritive (100 %) a été préparée en ajoutant dans 1000 L d'eau, les macro-nutriments suivants: HNO₃ (0,28 L), (NH₄)₂HPO₄ (120 g), Ca(NO₃)₂ (40 g), Mg(NO₃)₂ (140 g), KNO₃ (550 g), (NH₄)₂MoO₄ (0,05 g), H₃BO₃ (15 g), MnSO₄.H₂O (2 g), ZnSO₄.7H₂O (1 g), CuSO₄.5 H₂O (0,25 g), Sequestrene^{®} (10 g) (solution Fe-EDTA). Les graines de pois ont été mises à germer dans du sable humide pendant 6 jours, puis placées dans des trous d'un couvercle (35 trous/couvercle, 20 mm de diamètre) d'un pot en PVC opaque contenant la solution de culture hydroponique (47 L, pH 5,8).

Dans une première expérience, de l'acétone ou le composé à tester (dissous dans l'acétone) ont été ajoutés à la solution de culture hydroponique pour obtenir une concentration finale de 0 ou 1 µM de composé à tester et 0,01 % d'acétone.

La solution de culture hydroponique a été aérée continuellement au moyen d'une pompe d'aquarium et remplacée chaque semaine. 8 jours après le début du traitement, las longueurs de bourgeon/ ramifications (noeuds 1 à 6) ont été mesurées avec un pied à coulisse électronique.

Cette expérience a été réalisée pour les composés selon l'invention (IIa) et (IIIb), ainsi que le composé GR24 en tant que composé comparatif. Un témoin négatif (« CTRL 0 »), correspondant à un traitement avec le solvant seul, a également été réalisé.

Les résultats (données moyennes sur 20 plantes), en termes d'une part de longueur des ramifications aux noeuds 3 à 5, et d'autre part de hauteur de plante, sont montrés respectivement sur les Figures 11 et 12.

Ces résultats démontrent l'activité des composés conformes à l'invention (IIa) et (IIIb) sur l'ensemble des ramifications des noeuds 3 à 5. Un effet significatif sur la hauteur des plantes est également mis en évidence, comparable entre le composé comparatif GR24 et le composé conforme à l'invention (IIa), et plus important pour le composé conforme à l'invention (IIIb).

La même expérience a été réalisée pour le composé conforme à l'invention (II"g), et le composé comparatif GR24. Du diméthylsulfoxyde (DMSO) ou le composé à tester, dissous dans le DMSO, ont été ajoutés à la solution de culture hydroponique pour obtenir une concentration finale de 0 ou 3 µM de composé à tester et 0,01 % de DMSO.

Les résultats (données moyennes sur 10 à 12 plantes), en termes d'une part de longueur des ramifications aux noeuds 3 à 4, et d'autre part de hauteur de plante, sont montrés respectivement sur les Figures 13 et 14.

Ces résultats démontrent, pour le composé conforme à l'invention (II"g), une activité sur l'ensemble des ramifications des noeuds 3 à 4, ainsi qu'un effet sur la hauteur des plantes, qui sont tous deux supérieurs à ceux du composé comparatif GR24.

### EXEMPLE 6 - Etude de la toxicité chez le pois

Dans cet exemple, la toxicité des composés conformes à l'invention (IIa) et (IIIb), et du composé comparatif GR24, a été évaluée par dépôt au noeud 3 sur plantes mutantes *rms4* hyper ramifiées décrites dans la publication de Johnson et al. (1997) et ne répondant pas à l'action hormonale des strigolactones (lignée M3T-946 issue de la variété Térèse).

Le protocole mis en oeuvre est conforme à celui décrit dans l'Exemple 4 ci-avant. Des concentrations de 100 nM et 3 µM en composés ont été testées. Un contrôle négatif constitué du solvant seul (« CTL 0 ») a également été testé.

Le nombre de bourgeons morts suite au traitement par 3 µM de composé a également été déterminé après 8 jours de traitement (sur des groupes de 48 plantes).

Les résultats obtenus sont montrés, sur la Figure 15, pour la longueur de bourgeon/ramification au noeud 3, et sur la Figure 16, pour le nombre de bourgeons morts après traitement. Ces résultats mettent en évidence l'absence d'effets des composés conformes à l'invention (IIa) et (IIIb) par rapport au contrôle négatif, et entre les deux doses de traitement. De la même façon, aucune différence significative n'est remarquable sur le nombre de bourgeons morts suite au traitement. En particulier, le traitement par le composé (IIIb) n'a entraîné la mort d'aucun bourgeon.

### EXEMPLE 7 - Etude de l'activité germinative sur des plantes parasites

Dans cet exemple, l'activité germinative des composés conformes à l'invention (IIa) et (IIIb) et du composé comparatif GR24 a été évaluée sur les plantes parasites : *Orobanche cumana, Orobanche minor, Striga hermonthica, Phelipanche ramosa (pv T et pv C).*

Le protocole de test utilisé a été le suivant.

Deux populations de *Phelipanche ramosa* ont été utilisées dans cette étude, pathovar (pv) C and T, décrites dans la publication de Benharrat et al. (2005). Des graines de *Phelipanche ramosa* pathovar C ont été recueillies à Saint Martin de Fraigneau, France, sur des orobanches parasitant le colza d'hiver (*Brassica napus* L.) en 2005 et des graines de *Phelipanche ramosa* pathovars T dans la Sarthe, France, sur des orobanches se développant sur du chanvre (*Cannabis sativa* L.) en 2007.

Des graines *d'Orobanche cumana* Wallr. ont été collectées à partir d'orobanches parasitant le tournesol (*Helianthus annuus* L.), à Longeville-sur-mer, France, en 2009 ; des graines d'orobanche de trèfle (*Orobanche minor* Sm.) à partir de parasite de trèfle rouge (*Trifolium pratense* L.) ont été obtenues du Pr K. Yoneyama (Université d'Utsunomiya, Japon).

Des graines de *Striga hermonthica* (Del.) Benth ont été collectées à Gadarif, Est Soudan, en 1999.

Les graines sont stockées au sec et à l'obscurité à 25 °C.

Les composés à étudier ont été re-suspendus dans l'acétone à 10 mmol.L⁻¹, puis dilués avec de l'eau à 1 mmol.L⁻¹ (eau/acétone; v/v; 99/1). Des dilutions de 1×10⁻³ mol.L⁻¹ à 1×10⁻¹⁵ mol.L⁻¹ ont alors été réalisée dans un mélange eau/acétone (v/v; 99/1). Des graines de plantes parasites ont été stérilisées en surface selon le protocole décrit dans la publication de Vieira Dos Santos et al. (2003), puis re-suspendues dans de l'eau in stérile (10 g.L⁻¹) et distribuées sur une plaque à 96-puits (à raison de 50 µL, soit environ 100 graines par puits). Après préconditionnement (7 jours, 21 °C, dans l'obscurité, plaque fermée hermétiquement, excepté pour les graines de S. *hermonthica* qui ont été préconditionnées à 30 °C), les composés à étudier ont été ajoutés et les volumes ajustés à 100 µL avec de l'eau (eau/acétone; v/v; 999/1).

Dans une plaque, une gamme de concentrations de 10⁻¹³ mol.L⁻¹ à 10⁻⁶ mol.L⁻¹ a été appliquée pour GR24 et (IIa) et de 10⁻¹² à 10⁻⁵ mol.L⁻¹ pour (IIIb). Des contrôles ont été réalisés avec un mélange eau/acétone (v/v; 999/1) et sans graines.

Les plaques ont été incubées pour permettre la germination (21 °C, à l'obscurité ou 30 °C pourr *Striga hermonthica.*)*.* Après 4 jours, les graines ayant germé ont été comptées sous un microscope stéréo (SZX10, Olympus). Les graines ont été considérées comme ayant germé lorsque le radicule dépassait du tégument de la graine. Chaque test de germination a été répété au moins 3 fois. Pour chaque composé testé, des courbes dose-réponse (g = f(c), où g est le pourcentage de germination et c la concentration (mol.L⁻¹)), et la EC₅₀ (concentration efficace médiane) ont été modélisées à partir d'une courbe logistique à 4 paramètres calculée avec SigmaPlot^{®} 10.0.

Pour chacun des 3 composés testés, la concentration EC₅₀ pour chaque parasite ainsi obtenue est montrée sur la Figure 17. On y observe que les composés conformes à l'invention présentent une activité très faible, et bien inférieure à celle de GR24, sur la germination des graines de plantes parasites, et ceci sur les 4 parasites étudiés.

Les pourcentages de germination maximale induite par les composés testés pour chaque plante parasite ont été déterminés. Ces pourcentages sont donnés dans le Tableau 2 ci-après.

**Tableau 2 - Pourcentage de germination maximale induite par les composés pour différentes plantes parasites**

| | GR24 | (IIa) | (IIIb) |
|---|---|---|---|
| *Phelipanche ramosa pv C* | 89 ± 6 % | 27 ± 4 % | no |
| *Phelipanche ramosa pv T* | 87 ± 7 % | 72 ± 2 % | 88 ± 6 % |
| *Striga hermonthica* | 57 ± 5 % | no | no |
| *Orobanche minor* | 81 ± 5 % | no | 60 ± 6 % |
| *Orobanche cumana* | 78 ± 5 % | no | no |

| | | | |
|---|---|---|---|
| no : pas de germination significative par rapport au contrôle négatif (< 2 %) | | | |

Les composés conformes à l'invention présentent avantageusement une activité sur la germination des plantes parasites largement plus faible que le composé comparatif proposé par l'art antérieur GR24.

L'ensemble des résultats ci-avant démontre que les composés selon l'invention, et notamment les composés répondant aux formules (IIa) et (IIIb), possèdent une activité biologique pour le contrôle de la ramification qui est comparable à celle des strigolactones naturelles et aux analogues synthétiques de l'art antérieur GR24 et GR5, tout en étant bien plus faciles à préparer que ces analogues synthétiques et en présentant une cytotoxicité moindre. Ces composés selon l'invention permettent en outre de dissocier l'activité sur la ramification et celle sur la germination de l'orobanche et de *Striga hermonthica.*

La description ci-avant illustre clairement que par ses différentes caractéristiques et leurs avantages, la présente invention atteint les objectifs qu'elle s'était fixés. En particulier, elle fournit des composés simples à synthétiser, et qui présentent une activité biologique importante d'inhibition de la ramification chez les plantes supérieures, si bien que le traitement de telles plantes par ces composés permet de contrôler leur croissance et leur architecture de manière tout à fait optimale, au moyen qui plus est de faibles quantités de composé, en vue d'améliorer le rendement des cultures.

### REFERENCES BIBILOGRAPHIQUES

Benharrat et al. (2005) Virulence diversity among branched broomrape (o-ramosa I.) populations in France. Agron Sustainable Dev 25(1):123-128
Beveridge et al. (1997) The rms1 mutant of pea has elevated indole-3-acetic acid levels and reduced root-sap zeatin riboside content but increased branching controlled by graft-transmissible signal(s). Plant Physiol. 115: 1251-1258
Canevet et al. (1978) Friedel-Crafts Reaction of Aromatic Derivatives with 1,4-Dicarbonyl-2,3-Ethylenic Compounds .2. Alkylations by Some 5-Hydroxy or 5-Chloro-2,5-Dihydro-2-Furannones - New Method for Synthesis of 1h-Indene-1-Carboxylic Acids. Tetrahedron 34: 1935-1942
Dos Santos et al. (2003) Defense gene expression analysis of Arabidopsis thaliana parasitized by Orobanche ramosa. Phytopathology 93: 451-457
Fukui et al. (2011) New branching inhibitors and their potential as strigolactone mimics in rice. Bioorg. Med. Chem. Lett. 21: 4905-4908
Johnson et al. (1981) The Preparation of Synthetic Analogs of Strigol. Journal of the Chemical Society-Perkin Transactions 1: 1734-1743
Johnson et al. (2006) Branching genes are conserved across species. Genes controlling a novel signal in pea are coregulated by other long-distance signals. Plant Physiol. 142: 1014-1026
Mangnus et al. (1992) Improved Synthesis of Strigol Analog GR24 and Evaluation of the Biological-Activity of Its Diastereomers. J. Agric. Food Chem. 40: 1230-1235
Nair et al. (1981) Degenerative chemistry of malondialdehyde - structure, stereochemistry, and kinetics of formation of enaminals from reaction with amino-acids. J. Am. Chem. Soc. 103: 3030-3036
Timonen et al. (2011) Synthesis and anti-inflammatory effects of a series of novel 7-hydroxycoumarin derivatives. Eur. J. Med. Chem. 46: 3845-3850

## Revendications

1. Composé de formule générale (I) : 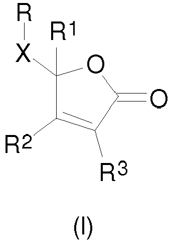 dans laquelle :
X représente un atome d'oxygène, un atome de soufre, NH ou un radical N-alkyl,
R¹ et R², identiques ou différents, représentent chacun un atome d'hydrogène ou un radical hydrocarboné linéaire ou ramifié, saturé ou insaturé, en C1-C10,
R¹ et R² ne représentant pas tous deux un atome d'hydrogène,
R³ représente un radical hydrocarboné linéaire ou ramifié, saturé ou insaturé, en C1-C10,
et R représente un radical phényle mono-substitué par un substituant Y choisi parmi Cl, Br, I et CF₃, ou un radical phényle di-substitué par un substituant Y et un substituant Z, Y et Z, identiques ou différents, étant choisis chacun parmi Cl, Br, I et CF₃, ou formant ensemble un cycle saturé ou insaturé ou aromatique, contenant le cas échéant un ou plusieurs hétéroatomes, et éventuellement substitué,
ou R représente un radical : 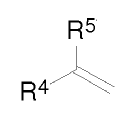
où R⁴ représente un radical hydrocarboné linéaire ou ramifié, saturé ou insaturé,
et R⁵ représente un radical hydrocarboné linéaire ou ramifié, saturé ou insaturé, le cas échéant substitué, un groupe COR⁶ ou un groupe CO₂R⁶, où R⁶ représente un atome d'hydrogène ou un radical hydrocarboné linéaire ou ramifié, saturé ou insaturé.

2. Composé selon la revendication 1, **caractérisé en ce que** R³ représente un radical alkyle linéaire en C1-C10, de préférence un radical méthyle.

3. Composé selon l'une des revendications 1 à 2, **caractérisé en ce que** R¹ et R² représentent chacun un atome d'hydrogène ou un radical alkyle linéaire ou ramifié en C1-C10.

4. Composé selon la revendication 3, **caractérisé en ce que** R¹ représente un atome d'hydrogène et R² représente un radical alkyle linéaire ou ramifié en C1-C10, de préférence un radical méthyle.

5. Composé selon l'une quelconque des revendications 1 à 4, **caractérisé en ce que** X représente un atome de soufre ou X représente un atome d'oxygène.

6. Composé selon l'une quelconque des revendications 1 à 5, **caractérisé en ce que** R représente un radical phényle substitué au moins en position para.

7. Composé selon l'une quelconque des revendications précédentes, de formule générale (II) : 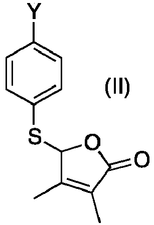 dans laquelle Y est choisi parmi Cl, Br, I et CF₃.

8. Composé selon la revendication 7, dans lequel Y est un atome de chlore.

9. Composé selon l'une quelconque des revendications 1 à 6, de formule générale (II") : 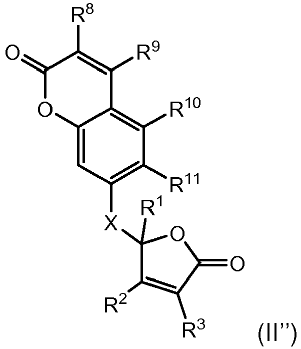 dans laquelle :
X représente un atome d'oxygène, un atome de soufre, NH ou un radical N-alkyl,
R¹ et R², identiques ou différents, représentent chacun un atome d'hydrogène ou un radical hydrocarboné linéaire ou ramifié, saturé ou insaturé, en C1-C10,
R¹ et R² ne représentant pas tous deux un atome d'hydrogène,
R³ représente un radical hydrocarboné linéaire ou ramifié, saturé ou insaturé, en C1-C10,
R⁸, R⁹, R¹⁰ et R¹¹, identiques ou différents, représentent chacun un atome d'hydrogène, Cl, Br, I, CF₃, CHO, CN, NO₂, un radical hydrocarboné linéaire ou ramifié, saturé ou insaturé, le cas échéant substitué, ou un groupement CO₂R¹², où R¹² représente un atome d'hydrogène ou un radical hydrocarboné linéaire ou ramifié, saturé ou insaturé, de préférence en C1-C10.

10. Composé selon la revendication 9, de formule générale (II") dans laquelle R⁸, R⁹, R¹⁰ et R¹¹ représentent chacun un atome d'hydrogène.

11. Composé selon l'une quelconque des revendications 1 à 5, **caractérisé en ce que** R représente un radical : 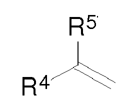
où R⁴ représente un radical alkyle ou un radical alkényle linéaires ou ramifiés en C1-C15,
et R⁵ représente un radical hydrocarboné linéaire ou ramifié en C1-C¹⁰, de préférence en C1-C5, le cas échéant substitué, un groupe COR⁶ ou un groupe CO₂R⁶, où R⁶ représente un radical hydrocarboné linéaire ou ramifié en C1-C10, de préférence en C1-C5.

12. Composé selon la revendication 11, dans lequel R⁵ représente un radical hydrocarboné linéaire ou ramifié, de préférence insaturé, en C1-C10, de préférence en C1-C5, substitué, préférentiellement à son extrémité libre, par un groupement électro-attracteur choisi de préférence parmi : CHO, CN, NO₂ et CO₂R⁷, où R⁷ représente un radical hydrocarboné linéaire ou ramifié en C1-C10, de préférence en C1-C5.

13. Composition pour le traitement des plantes supérieures, comprenant un composé selon l'une quelconque des revendications 1 à 12.

14. Composition selon la revendication 13, **caractérisée en ce que** la concentration en ledit composé est comprise entre 0,1 et 1000 nM, de préférence entre 1 et 100 nM.

15. Utilisation d'un composé selon l'une quelconque des revendications 1 à 12 pour le traitement d'une plante supérieure en vue de contrôler la croissance et l'architecture de ladite plante.

16. Utilisation selon la revendication 15, **caractérisée en ce qu'**on place au contact de la plante une quantité adaptée dudit composé de manière à inhiber la formation d'au moins une ramification.

17. Utilisation selon la revendication 16, **caractérisée en ce qu'**on applique une composition comprenant ledit composé sur une portion au moins partielle de la partie aérienne de la plante.

18. Utilisation selon l'une des revendications 16 à 17, **caractérisée en ce qu'**on applique une composition comprenant ledit composé sur des bourgeons axillaires de la plante.

19. Utilisation selon la revendication 16, **caractérisée en ce qu'**on injecte une composition comprenant ledit composé dans une partie aérienne de ladite plante.

20. Utilisation selon la revendication 16, **caractérisée en ce qu'**on apporte une composition comprenant ledit composé par au moins une racine de ladite plante.

21. Utilisation selon l'une quelconque des revendications 17 à 20, **caractérisée en ce que** la concentration en ledit composé dans ladite composition est comprise entre 0,1 et 1000 nM, de préférence entre 1 et 100 nM.

## Patentansprüche

1. Verbindung der allgemeinen Formel (I): 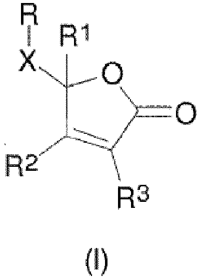 worin:
X für ein Sauerstoffatom, ein Schwefelatom, NH oder einen Rest N-Alkyl steht,
R¹ oder R², die gleich oder verschieden sind, jeweils für ein Wasserstoffatom oder einen geraden oder verzweigten, gesättigten oder ungesättigten C1-C10-Kohlenwasserstoffrest stehen,
R¹ und R² nicht beide für ein Wasserstoffatom stehen,
R³ für einen geraden oder verzweigten, gesättigten oder ungesättigten C1-C10-Kohlenwasserstoffrest steht und R für einen Phenylrest steht, der mit einem Substituenten Y, ausgewählt aus Cl, Br, I und CF₃, monosubstituiert ist oder für einen Phenylrest, der mit einem Substituenten Y und einem Substituenten Z disubstituiert ist, wobei Y und Z gleich oder verschieden sind und jeweils aus Cl, Br, I und CF₃ ausgewählt sind oder zusammen einen gesättigten oder ungesättigten oder aromatischen Ring bilden, der gegebenenfalls ein oder mehrere Heteroatome enthält und gegebenenfalls substituiert ist,
oder R für einen Rest: 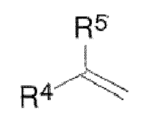
steht, wobei R⁴ für einen geraden oder verzweigten, gesättigten oder ungesättigten Kohlenwasserstoffrest steht
und R⁵ für einen geraden oder verzweigten, gesättigten oder ungesättigten, gegebenenfalls substituierten Kohlenwasserstoffrest, eine Gruppe COR⁶ oder eine Gruppe CO₂R⁶ steht, wobei R⁶ für ein Wasserstoffatom oder einen geraden oder verzweigten, gesättigten oder ungesättigten Kohlenwasserstoffrest steht.

2. Verbindung nach Anspruch 1, **dadurch gekennzeichnet, dass** R³ für einen geraden C1-C10-Alkylrest, vorzugsweise einen Methylrest, steht.

3. Verbindung nach einem der Ansprüche 1 bis 2, **dadurch gekennzeichnet, dass** R¹ und R² jeweils für ein Wasserstoffatom oder einen geraden oder verzweigten C1-C10-Alkylrest stehen.

4. Verbindung nach Anspruch 3, **dadurch gekennzeichnet, dass** R¹ für ein Wasserstoffatom steht und R² für einen geraden oder verzweigten C1-C10-Alkylrest, vorzugsweise einen Methylrest, steht.

5. Verbindung nach einem der Ansprüche 1 bis 4, **dadurch gekennzeichnet, dass** X für ein Schwefelatom steht oder X für ein Sauerstoffatom steht.

6. Verbindung nach einem der Ansprüche 1 bis 5, **dadurch gekennzeichnet, dass** R für einen zumindest in para-Stellung substituierten Phenylrest steht.

7. Verbindung nach einem der vorhergehenden Ansprüche der allgemeinen Formel (II): 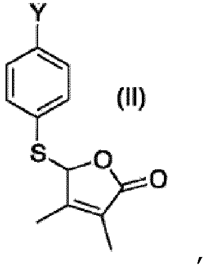 worin Y aus Cl, Br, I und CF₃ ausgewählt ist.

8. Verbindung nach Anspruch 7, worin Y ein Chloratom ist.

9. Verbindung nach einem der Ansprüche 1 bis 6 der allgemeinen Formel (II''): 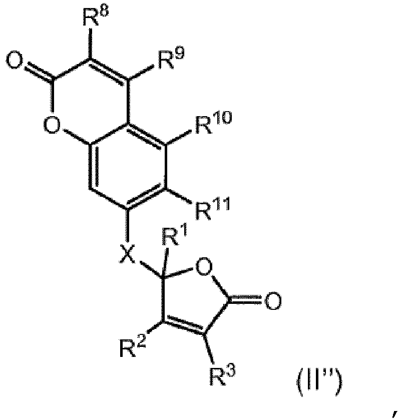 worin:
X für ein Sauerstoffatom, ein Schwefelatom, NH oder einen Rest N-Alkyl steht,
R¹ oder R², die gleich oder verschieden sind, jeweils für ein Wasserstoffatom oder einen geraden oder verzweigten, gesättigten oder ungesättigten C1-C10-Kohlenwasserstoffrest stehen,
R¹ und R² nicht beide für ein Wasserstoffatom stehen,
R³ für einen geraden oder verzweigten, gesättigten oder ungesättigten C1-C10-Kohlenwasserstoffrest steht,
R⁸, R⁹, R¹⁰ und R¹¹, die gleich oder verschieden sind, jeweils für ein Wasserstoffatom, Cl, Br, I, CF₃, CHO, CN, NO₂, einen geraden oder verzweigten, gesättigten oder ungesättigten, gegebenenfalls substituierten Kohlenwasserstoffrest oder eine Gruppe CO₂R¹² stehen, wobei R¹² für ein Wasserstoffatom oder einen geraden oder verzweigten, gesättigten oder ungesättigten, vorzugsweise C1-C10-, Kohlenwasserstoffrest steht.

10. Verbindung nach Anspruch 9 der allgemeinen Formel (II''), worin R⁸, R⁹, R¹⁰ und R¹¹ jeweils für ein Wasserstoffatom stehen.

11. Verbindung nach einem der Ansprüche 1 bis 5, **dadurch gekennzeichnet, dass** R für folgenden Rest steht: 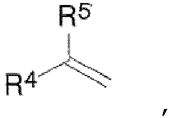
wobei R⁴ für einen geraden oder verzweigten C1-C15-Alkyl- oder Alkenylrest steht,
und R⁵ für einen geraden oder verzweigten C1-C10-, vorzugsweise C1-C5-, Kohlenwasserstoffrest, der gegebenenfalls substituiert ist, eine Gruppe COR⁶ oder eine Gruppe CO₂R⁶ steht, wobei R⁶ für einen geraden oder verzweigten C1-C10-, vorzugsweise C1-C5-, Kohlenwasserstoffrest steht.

12. Verbindung nach Anspruch 11, wobei R⁵ für einen geraden oder verzweigten, vorzugsweise ungesättigten, C1-C10-, vorzugsweise C1-C5-, Kohlenwasserstoffrest steht, der vorzugsweise an seinem freien Ende mit einer elektronenanziehenden Gruppe substituiert ist, vorzugsweise ausgewählt aus: CHO, CN, NO₂ und CO₂R⁷, wobei R⁷ für einen geraden oder verzweigten C1-C10-, vorzugsweise C1-C5-, Kohlenwasserstoffrest steht.

13. Zusammensetzung zur Behandlung höherer Pflanzen, umfassend eine Verbindung nach einem der Ansprüche 1 bis 12.

14. Zusammensetzung nach Anspruch 13, **dadurch gekennzeichnet, dass** die Konzentration der Verbindung zwischen 0,1 und 1000 nM, vorzugsweise zwischen 1 und 100 nM, beträgt.

15. Verwendung einer Verbindung nach einem der Ansprüche 1 bis 12 zur Behandlung einer höheren Pflanze um das Wachstum und den Bau dieser Pflanze zu steuern.

16. Verwendung nach Anspruch 15, **dadurch gekennzeichnet, dass** man eine angemessene Menge der Verbindung mit der Pflanze in Kontakt bringt, um die Bildung mindestens einer Verzweigung zu verhindern.

17. Verwendung nach Anspruch 16, **dadurch gekennzeichnet, dass** man eine die Verbindung umfassende Zusammensetzung auf einen zumindest partiellen Abschnitt des oberirdischen Teils der Pflanze aufbringt.

18. Verwendung nach einem der Ansprüche 16 bis 17, **dadurch gekennzeichnet, dass** man eine die Verbindung umfassende Zusammensetzung auf die Achselknospen der Pflanze aufbringt.

19. Verwendung nach Anspruch 16, **dadurch gekennzeichnet, dass** man eine die Verbindung umfassende Zusammensetzung in einen oberirdischen Teil der Pflanze injiziert.

20. Verwendung nach Anspruch 16, **dadurch gekennzeichnet, dass** man eine die Verbindung umfassende Zusammensetzung durch mindestens eine Wurzel der Pflanze einbringt.

21. Verwendung nach einem der Ansprüche 17 bis 20, **dadurch gekennzeichnet, dass** die Konzentration der Verbindung in der Zusammensetzung zwischen 0,1 und 1000 nM, vorzugsweise zwischen 1 und 100 nM, beträgt.

## Claims

1. A compound of general formula (I): 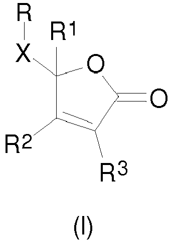 in which:
X represents an oxygen atom, a sulfur atom, NH or an N-alkyl radical,
R¹ and R², which may be identical or different, each represent a hydrogen atom or a linear or branched, saturated or unsaturated, C₁-C₁₀ hydrocarbon-based radical,
R¹ and R² not both representing a hydrogen atom,
R³ represents a linear or branched, saturated or unsaturated, C₁-C₁₀ hydrocarbon-based radical,
and R represents a phenyl radical monosubstituted with a substituent Y chosen from Cl, Br, I and CF₃, or a phenyl radical disubstituted with a substituent Y and a substituent Z, Y and Z, which may be identical or different, being each chosen from Cl, Br, I and CF₃, or forming together a saturated or unsaturated or aromatic, optionally substituted, ring which may contain one or more heteroatoms,
or R represents a radical: 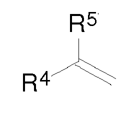
where R⁴ represents a linear or branched, saturated or unsaturated, hydrocarbon-based radical,
and R⁵ represents a linear or branched, saturated or unsaturated, hydrocarbon-based radical, optionally substituted, a COR⁶ group or a CO₂R⁶ group, where R⁶ represents a hydrogen atom or a linear or branched, saturated or unsaturated, hydrocarbon-based radical.

2. The compound as claimed in claim 1, **characterized in that** R³ represents a linear C₁-C₁₀ alkyl radical, preferably a methyl radical.

3. The compound as claimed in either of claims 1 and 2, **characterized in that** R¹ and R² each represent a hydrogen atom or a linear or branched C₁-C₁₀ alkyl radical.

4. The compound as claimed in claim 3, **characterized in that** R¹ represents a hydrogen atom and R² represents a linear or branched C₁-C₁₀ alkyl radical, preferably a methyl radical.

5. The compound as claimed in any one of claims 1 to 4, **characterized in that** X represents a sulfur atom or X represents an oxygen atom.

6. The compound as claimed in any one of claims 1 to 5, **characterized in that** R represents a phenyl radical substituted at least in the para-position.

7. The compound as claimed in any one of the preceding claims, of general formula (II): 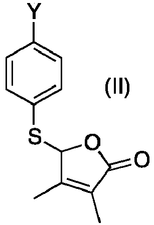 in which Y is chosen from Cl, Br, I and CF₃.

8. The compound as claimed in claim 7, in which Y is a chlorine atom.

9. The compound as claimed in any one of claims 1 to 6, of general formula (II"): 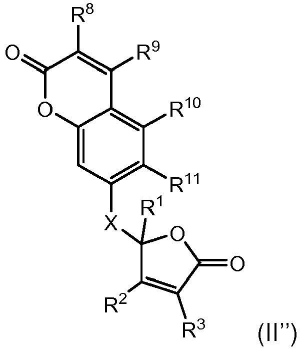 in which:
X represents an oxygen atom, a sulfur atom, NH or an N-alkyl radical,
R¹ and R², which may be identical or different, each represent a hydrogen atom or a linear or branched, saturated or unsaturated, C₁-C₁₀ hydrocarbon-based radical,
R¹ and R² not both representing a hydrogen atom,
R³ represents a linear or branched, saturated or unsaturated, C₁-C₁₀ hydrocarbon-based radical,
R⁸, R⁹, R¹⁰ and R¹¹, which may be identical or different, each represent a hydrogen atom, Cl, Br, I, CF₃, CHO, CN, NO₂, a linear or branched, saturated or unsaturated, optionally substituted, hydrocarbon-based radical, or a CO₂R¹² group, where R¹² represents a hydrogen atom or a linear or branched, saturated or unsaturated, preferably C₁-C₁₀, hydrocarbon-based radical.

10. The compound as claimed in claim 9, of general formula (II") in which R⁸, R⁹, R¹⁰ and R¹¹ each represent a hydrogen atom.

11. The compound as claimed in any one of claims 1 to 5, **characterized in that** R represents a radical: 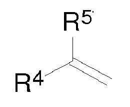
where R⁴ represents a linear or branched C₁-C₁₅ alkyl radical or alkenyl radical,
and R⁵ represents a linear or branched, C₁-C₁₀, preferably C₁-C₅, optionally substituted, hydrocarbon-based radical, a COR⁶ group or a CO₂R⁶ group, where R⁶ represents a linear or branched, C₁-C₁₀, preferably C₁-C₅, hydrocarbon-based radical.

12. The compound as claimed in claim 11, in which R⁵ represents a linear or branched, preferably unsaturated, C₁-C₁₀, preferably C₁-C₅, hydrocarbon-based radical, which is substituted, preferentially at its free end, with an electron-withdrawing group preferably chosen from : CHO, CN, NO₂ and CO₂R⁷, where R⁷ represents a linear or branched, C₁-C₁₀, preferably C₁-C₅, hydrocarbon-based radical.

13. A composition for the treatment of higher plants, comprising a compound as claimed in any one of claims 1 to 12.

14. The composition as claimed in claim 13, **characterized in that** the concentration of said compound is between 0.1 and 1000 nM, preferably between 1 and 100 nM.

15. The use of a compound as claimed in any one of claims 1 to 12, for the treatment of a higher plant with a view to controlling the growth and the architecture of said plant.

16. The use as claimed in claim 15, **characterized in that** an appropriate amount of said compound is brought into contact with the plant so as to inhibit the formation of at least one branch.

17. The use as claimed in claim 16, **characterized in that** a composition comprising said compound is applied to an at least partial portion of the aerial part of the plant.

18. The use as claimed in either of claims 16 and 17, **characterized in that** a composition comprising said compound is applied to axillary buds of the plant.

19. The use as claimed in claim 16, **characterized in that** a composition comprising said compound is injected into an aerial part of said plant.

20. The use as claimed in claim 16, **characterized in that** a composition comprising said compound is introduced via at least one root of said plant.

21. The use as claimed in any one of claims 17 to 20, **characterized in that** the concentration of said compound in said composition is between 0.1 and 1000 nM, preferably between 1 and 100 nM.
